(19) Europäisches Patentamt
European Patent Office
Office européen des brevets

(11) EP 3 932 691 A1

(12) # EUROPEAN PATENT APPLICATION
published in accordance with Art. 153(4) EPC

(43) Date of publication:
**05.01.2022 Bulletin 2022/01**

(21) Application number: **20762179.8**

(22) Date of filing: **21.02.2020**

(51) Int Cl.:
***B60C 1/00*** *(2006.01)* ***C08K 5/372*** *(2006.01)*
***C08K 5/378*** *(2006.01)* ***C08L 21/00*** *(2006.01)*

(86) International application number:
**PCT/JP2020/006962**

(87) International publication number:
**WO 2020/175344 (03.09.2020 Gazette 2020/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.02.2019 JP 2019034798**

(71) Applicant: **SUMITOMO CHEMICAL COMPANY LIMITED**
**Chuo-ku**
**Tokyo 104-8260 (JP)**

(72) Inventors:
- **KAMIMOTO, Natsuyo**
  **Ichihara-shi, Chiba 299-0195 (JP)**
- **HARA, Takeshi**
  **Ichihara-shi, Chiba 299-0195 (JP)**
- **NOJIMA, Shiki**
  **Ichihara-shi, Chiba 299-0195 (JP)**

(74) Representative: **Vossius & Partner**
**Patentanwälte Rechtsanwälte mbB**
**Siebertstraße 3**
**81675 München (DE)**

(54) **VULCANIZED RUBBER COMPOSITION**

(57) The present invention provides a vulcanized rubber composition which comprises a compound represented by formula (I) and a vulcanized rubber and in which the content of the compound represented by formula (I) is 0.00005 to 5 % by weight with respect to the entire vulcanized rubber composition (the groups in formula (I) are as defined in the description).

$R^{4a}$, $X^{3a}$, $X^{5a}$, SH, $X^{1a}$, $R^{6a}$ (I)

EP 3 932 691 A1

## Description

### Technical Field

The present invention relates to a vulcanized rubber composition.

### Background Art

Vulcanized rubber compositions are used in various fields (e.g., tires), and techniques for improving the physical properties thereof have been proposed. For example, Patent Literature 1 discloses use of pyrimidine derivatives (especially 2,2-bis(4,6-dimethylpyrimidyl)disulfide) in order to achieve hardness stabilization of a vulcanized rubber composition (in detail, suppression of increase in the hardness due to aging of a vulcanized rubber composition).

### Citation List

### Patent Literature

Patent Literature 1: Japanese Translation of PCT International Application Publication No. 2004-500471

### Summary of Invention

### Technical Problem

Among fields in which vulcanized rubber compositions are used, the field of tires is important. Abrasion resistance is one of important performance capabilities in tires, and the abrasion resistance of vulcanized rubber compositions for use in tires is required to be improved. The present invention has been made in view of the situation, and an object thereof is to provide a vulcanized rubber composition excellent in abrasion resistance.

### Solution to Problem

The present invention that can achieve the above-described object is as follows.

[1] A vulcanized rubber composition comprising a compound represented by the formula (I):

[Formula 1]

(I)

[wherein,

$X^{1a}$ represents a nitrogen atom or C-$R^{1a}$,
$X^{3a}$ represents a nitrogen atom or C-$R^{3a}$,
$X^{5a}$ represents a nitrogen atom or C-$R^{5a}$,
at least one of $X^{1a}$, $X^{3a}$, and $X^{5a}$ is a nitrogen atom, and
$R^{1a}$ and $R^{3a}$ to $R^{6a}$ each independently represent a hydrogen atom, a halogen atom, a $C_{1-18}$ alkyl group optionally having a substituent, a $C_{3-10}$ cycloalkyl group optionally having a substituent, a $C_{6-18}$ aryl group optionally having a substituent, a $C_{7-20}$ aralkyl group optionally having a substituent, a carboxy group, a $C_{1-18}$ alkoxy-carbonyl group optionally having a substituent, a $C_{3-10}$ cycloalkyloxy-carbonyl group optionally having a substituent, a $C_{6-18}$ aryloxy-carbonyl group optionally having a substituent, a $C_{7-20}$ aralkyloxy-carbonyl group optionally having a substituent, a carbamoyl group optionally having a substituent, a hydroxy group, a $C_{1-18}$ alkoxy group optionally having a substituent, a $C_{3-10}$ cycloalkyloxy group optionally having a substituent, a $C_{6-18}$ aryloxy group optionally

having a substituent, a $C_{7-20}$ aralkyloxy group optionally having a substituent, a $C_{1-18}$ alkyl-carbonyloxy group optionally having a substituent, a $C_{3-10}$ cycloalkyl-carbonyloxy group optionally having a substituent, a $C_{6-18}$ aryl-carbonyloxy group optionally having a substituent, a $C_{7-20}$ aralkyl-carbonyloxy group optionally having a substituent, an amino group optionally having a substituent, or a nitro group.] and a vulcanized rubber, wherein a content of the compound represented by the formula (I) is 0.00005 to 5% by weight with respect to the entire vulcanized rubber composition.

[2] The vulcanized rubber composition according to [1], wherein one or two of $X^{1a}$, $X^{3a}$, and $X^{5a}$ are nitrogen atoms.
[3] The vulcanized rubber composition according to [1] or [2], wherein $R^{1a}$ and $R^{3a}$ to $R^{6a}$ are each independently a hydrogen atom, a $C_{1-18}$ alkyl group optionally having a substituent, a carboxy group, a $C_{1-18}$ alkoxy-carbonyl group optionally having a substituent, or a nitro group.
[4] The vulcanized rubber composition according to [3], wherein the $C_{1-18}$ alkyl group optionally having a substituent is a $C_{1-12}$ alkyl group optionally having a halogen atom, more preferably a $C_{1-6}$ alkyl group optionally having a halogen atom, and further preferably a $C_{1-3}$ alkyl group optionally having a halogen atom.
[5] The vulcanized rubber composition according to [3] or [4], wherein the $C_{1-18}$ alkoxy-carbonyl group optionally having a substituent is a $C_{1-12}$ alkoxy-carbonyl group and more preferably a $C_{1-12}$ alkoxy-carbonyl group.
[6] The vulcanized rubber composition according to [1], wherein the compound represented by the formula (I) is at least one selected from the group consisting of:

a compound (Ia), wherein both $X^{1a}$ and $X^{3a}$ are nitrogen atoms, $X^{5a}$ is C-$R^{5a}$, and $R^{4a}$ to $R^{6a}$ are each independently a hydrogen atom, a $C_{1-18}$ alkyl group optionally having a substituent, or a nitro group,
a compound (Ib), wherein $X^{1a}$ is a nitrogen atom, $X^{3a}$ is C-$R^{3a}$, $X^{5a}$ is C-$R^{5a}$, and $R^{3a}$ to $R^{6a}$ is each independently a hydrogen atom, a $C_{1-18}$ alkyl group optionally having a substituent, or a nitro group, and
a compound (Ic), wherein $X^{1a}$ is C-$R^{1a}$, $X^{3a}$ is C-$R^{3a}$, $X^{5a}$ is a nitrogen atom, and $R^{1a}$, $R^{3a}$, $R^{4a}$, and $R^{6a}$ are each independently a hydrogen atom, a $C_{1-18}$ alkyl group optionally having a substituent, or a nitro group.

[7] The vulcanized rubber composition according to [6], wherein, in the compound (Ia), $R^{4a}$ to $R^{6a}$ are each independently a hydrogen atom or a $C_{1-18}$ alkyl group optionally having a substituent.
[8] The vulcanized rubber composition according to [6], wherein, in the compound (Ia), $R^{4a}$ and $R^{6a}$ are each independently a $C_{1-18}$ alkyl group optionally having a substituent, and $R^{5a}$ is a hydrogen atom.
[9] The vulcanized rubber composition according to any one of [6] to [8], wherein, in the compound (Ib), $R^{3a}$, $R^{4a}$, and $R^{6a}$ are each independently a hydrogen atom or a $C_{1-18}$ alkyl group optionally having a substituent, and are more preferably all hydrogen atoms.
[10] The vulcanized rubber composition according to any one of [6] to [9], wherein, in the compound (Ib), $R^{5a}$ is a hydrogen atom or a nitro group.
[11] The vulcanized rubber composition according to any one of [6] to [10], wherein, in the compound (Ic), $R^{1a}$, $R^{3a}$, $R^{4a}$, and $R^{6a}$ each independently a hydrogen atom or a $C_{1-18}$ alkyl group optionally having a substituent, and are more preferably all hydrogen atoms.
[12] The vulcanized rubber composition according to [1], wherein

both $X^{1a}$ and $X^{3a}$ are nitrogen atoms,
$X^{5a}$ is C-$R^{5a}$,
$R^{4a}$ and $R^{6a}$ are each independently a $C_{1-18}$ alkyl group optionally having a substituent, and
$R^{5a}$ is a hydrogen atom.

[13] The vulcanized rubber composition according to any one of [3] to [12], wherein the $C_{1-18}$ alkyl group optionally having a substituent is a $C_{1-18}$ alkyl group, more preferably a $C_{1-12}$ alkyl group, further preferably a $C_{1-6}$ alkyl group, and particularly preferably a $C_{1-3}$ alkyl group.
[14] The vulcanized rubber composition according to [1], wherein
the compound represented by the formula (I) is at least one selected from the group consisting of compounds represented by any of the following formula (Ia-1) to formula (Ic-1):

[Formula 2]

(Ia-1) (Ia-2) (Ib-1) (Ib-2) (Ic-1)

and preferably a compound represented by the above formula (Ia-1).

[15] The vulcanized rubber composition according to any one of [1] to [14], wherein the content of the compound represented by the formula (I) is 0.00008% by weight or more, more preferably 0.00010% by weight or more, further preferably 0.0010% by weight or more, and particularly preferably 0.010% by weight or more with respect to the entire vulcanized rubber composition.

[16] The vulcanized rubber composition according to any one of [1] to [15], wherein the content of the compound represented by the formula (I) is 3% by weight or less and more preferably 1% by weight or less with respect to the entire vulcanized rubber composition.

[17] The vulcanized rubber composition according to any one of [1] to [16], further comprising a compound represented by the formula (II):

[Formula 3]

(II)

[wherein,

$X^{1b}$ represents a nitrogen atom or C-$R^{1b}$,
$X^{3b}$ represents a nitrogen atom or C-$R^{3b}$,
$X^{5b}$ represents a nitrogen atom or C-$R^{5b}$,
$X^{1c}$ represents a nitrogen atom or C-$R^{1c}$,
$X^{3c}$ represents a nitrogen atom or C-$R^{3c}$,
$X^{5c}$ represents a nitrogen atom or C-$R^{5c}$,
at least one of $X^{1b}$, $X^{3b}$, $X^{5b}$, $X^{1c}$, $X^{3c}$, and $X^{5c}$ is a nitrogen atom, and
$R^{1b}$ and $R^{3b}$ to $R^{6b}$ and $R^{1c}$ and $R^{3c}$ to $R^{6c}$ each independently represent a hydrogen atom, a halogen atom, a $C_{1-18}$ alkyl group optionally having a substituent, a $C_{3-10}$ cycloalkyl group optionally having a substituent, a $C_{6-18}$ aryl group optionally having a substituent, a $C_{7-20}$ aralkyl group optionally having a substituent, a carboxy group, a $C_{1-18}$ alkoxy-carbonyl group optionally having a substituent, a $C_{3-10}$ cycloalkyloxy-carbonyl group optionally having a substituent, a $C_{6-18}$ aryloxy-carbonyl group optionally having a substituent, a $C_{7-20}$ aralkyloxy-carbonyl group optionally having a substituent, a carbamoyl group optionally having a substituent, a hydroxy group, a $C_{1-18}$ alkoxy group optionally having a substituent, a $C_{3-10}$ cycloalkyloxy group optionally having a substituent, a $C_{6-18}$ aryloxy group optionally having a substituent, a $C_{7-20}$ aralkyloxy group optionally having a substituent, a $C_{1-18}$ alkyl-carbonyloxy group optionally having a substituent, a $C_{3-10}$ cycloalkyl-carbonyloxy group optionally having a substituent, a $C_{6-18}$ aryl-carbonyloxy group optionally having a substituent, a $C_{7-20}$ aralkyl-carbonyloxy group optionally having a substituent, an amino group optionally having a substituent, or a nitro group] at a content of 0.0001 to 1.0% by weight with respect to the entire vulcanized rubber composition.

[18] The vulcanized rubber composition according to [17], wherein one or more and five or less of $X^{1b}$, $X^{3b}$, $X^{5b}$, $X^{1c}$, $X^{3c}$, and $X^{5c}$ are nitrogen atoms.

[19] The vulcanized rubber composition according to [17] or [18], wherein both $X^{1b}$ and $X^{1c}$ are nitrogen atoms, or

$X^{1b}$ is C-$R^{1b}$ and $X^{1c}$ is C-$R^{1c}$.

[20] The vulcanized rubber composition according to [19], wherein $R^{1b}$ and $R^{1c}$ are the same.

[21] The vulcanized rubber composition according to any one of [17] to [20], wherein both $X^{3b}$ and $X^{3c}$ are nitrogen atoms, or $X^{3b}$ is C-$R^{3b}$ and $X^{3c}$ is C-$R^{3c}$.

[22] The vulcanized rubber composition according to [21], wherein $R^{3b}$ and $R^{3c}$ are the same.

[23] The vulcanized rubber composition according to any one of [17] to [22], wherein both $X^{5b}$ and $X^{5c}$ are nitrogen atoms, or $X^{5b}$ is C-$R^{5b}$ and $X^{5c}$ is C-$R^{5c}$.

[24] The vulcanized rubber composition according to [23], wherein $R^{5b}$ and $R^{5c}$ are the same.

[25] The vulcanized rubber composition according to any one of [17] to [24], wherein $R^{1b}$ and $R^{3b}$ to $R^{6b}$ and $R^{1c}$ and $R^{3c}$ to $R^{6c}$ are each independently a hydrogen atom, a $C_{1-18}$ alkyl group optionally having a substituent, a carboxy group, a $C_{1-18}$ alkoxy-carbonyl group optionally having a substituent, or a nitro group.

[26] The vulcanized rubber composition according to [25], wherein the $C_{1-18}$ alkyl group optionally having a substituent is a $C_{1-12}$ alkyl group optionally having a halogen atom, more preferably a $C_{1-6}$ alkyl group optionally having a halogen atom, and further preferably a $C_{1-3}$ alkyl group optionally having a halogen atom.

[27] The vulcanized rubber composition according to [25] or [26], wherein the $C_{1-18}$ alkoxy-carbonyl group optionally having a substituent is a $C_{1-12}$ alkoxy-carbonyl group optionally having a substituent and more preferably a $C_{1-12}$ alkoxy-carbonyl group.

[28] The vulcanized rubber composition according to [17], wherein

the compound represented by the formula (II) is at least one selected from the group consisting of:

a compound (IIa), wherein $X^{1b}$, $X^{3b}$, $X^{1c}$, and $X^{3c}$ are all nitrogen atoms, $X^{5b}$ is C-$R^{5b}$, $X^{5c}$ is C-$R^{5c}$, and $R^{4b}$ to $R^{6b}$ and $R^{4c}$ to $R^{6c}$ are each independently a hydrogen atom, a $C_{1-18}$ alkyl group optionally having a substituent, or a nitro group,

a compound (IIb), wherein both $X^{1b}$ and $X^{1c}$ are nitrogen atoms, $X^{3b}$ is C-$R^{3b}$, $X^{5b}$ is C-$R^{5b}$, $X^{3c}$ is C-$R^{3c}$, $X^{5c}$ is C-$R^{5c}$, and $R^{3b}$ to $R^{6b}$ and $R^{3c}$ to $R^{6c}$ are each independently a hydrogen atom, a $C_{1-18}$ alkyl group optionally having a substituent, or a nitro group, and

a compound (IIc), wherein $X^{1b}$ is C-$R^{1b}$, $X^{3b}$ is C-$R^{3b}$, $X^{1c}$ is C-$R^{1c}$, $X^{3c}$ is C-$R^{3c}$, both $X^{5b}$ and $X^{5c}$ are nitrogen atoms, and $R^{1b}$, $R^{3b}$, $R^{4b}$, $R^{6b}$, $R^{1c}$, $R^{3c}$, $R^{4c}$, and $R^{6c}$ are each independently a hydrogen atom, a $C_{1-18}$ alkyl group optionally having a substituent, or a nitro group.

[29] The vulcanized rubber composition according to [28], wherein, in the compound (IIa), $R^{4b}$ to $R^{6b}$ and $R^{4c}$ to $R^{6c}$ are each independently a hydrogen atom or a $C_{1-18}$ alkyl group optionally having a substituent.

[30] The vulcanized rubber composition according to [28], wherein, in the compound (IIa), $R^{4b}$, $R^{6b}$, $R^{4c}$, and $R^{6c}$ are each independently a $C_{1-18}$ alkyl group optionally having a substituent, and both $R^{5b}$ and $R^{5c}$ are hydrogen atoms.

[31] The vulcanized rubber composition according to any one of [28] to [30], wherein, in the compound (IIb) , $R^{3b}$, $R^{4b}$, $R^{6b}$, $R^{3c}$, $R^{4c}$, and $R^{6c}$ are each independently a hydrogen atom or a $C_{1-18}$ alkyl group optionally having a substituent and more preferably all hydrogen atoms.

[32] The vulcanized rubber composition according to any one of [28] to [31], wherein, in compound (IIb) , $R^{5b}$ and $R^{5c}$ are a hydrogen atom or a nitro group.

[33] The vulcanized rubber composition according to any one of [28] to [32], wherein, in the compound (IIc), $R^{1b}$, $R^{3b}$, $R^{4b}$, $R^{6b}$, $R^{1c}$, $R^{3c}$, $R^{4c}$, and $R^{6c}$ are each independently a hydrogen atom or a $C_{1-18}$ alkyl group optionally having a substituent and more preferably all hydrogen atoms.

[34] The vulcanized rubber composition according to [17], wherein

$X^{1b}$, $X^{3b}$, $X^{1c}$, and $X^{3c}$ are all nitrogen atoms,
$X^{5b}$ is C-$R^{5b}$,
$X^{5c}$ is C-$R^{5c}$,
$R^{4b}$, $R^{6b}$, $R^{4c}$, and $R^{6c}$ are each independently a $C_{1-18}$ alkyl group optionally having a substituent, and
both $R^{5b}$ and $R^{5c}$ are hydrogen atoms.

[35] The vulcanized rubber composition according to any one of [17] to [34], wherein the $C_{1-18}$ alkyl group optionally having a substituent is a $C_{1-18}$ alkyl group, more preferably a $C_{1-12}$ alkyl group, further preferably a $C_{1-6}$ alkyl group, and particularly preferably a $C_{1-3}$ alkyl group.

[36] The vulcanized rubber composition according to [17], wherein the compound represented by the formula (II) is at least one selected from the group consisting of compounds represented by any of the following formula (IIa-1) to formula (IIc-1) :

[Formula 4]

(IIa-1) (IIa-2) (IIb-1) (IIb-2) (IIc-1)

and preferably a compound represented by the above formula (IIa-1) .

[37] The vulcanized rubber composition according to any one of [17] to [36], wherein the content of the compound represented by the formula (II) is 0.0001% by weight or more, more preferably 0.0002% by weight or more, further preferably 0.0005% by weight or more, and particularly preferably 0.0010% by weight or more with respect to the entire vulcanized rubber composition.

[38] The vulcanized rubber composition according to any one of [17] to [37], wherein the content of the compound represented by the formula (II) is 1.0% by weight or less, more preferably 0.5% by weight or less, further preferably 0.3% by weight or less, particularly preferably 0.2% by weight or less, and most preferably 0.1% by weight or less with respect to the entire vulcanized rubber composition.

[39] The vulcanized rubber composition according to any one of [1] to [38], wherein the vulcanized rubber is a rubber component crosslinked by a sulfur component.

[40] The vulcanized rubber composition according to [39], wherein the amount of the sulfur component is 0.1 to 10 parts by weight, more preferably 0.1 to 7 parts by weight, and further preferably 0.1 to 4 parts by weight per 100 parts by weight of the rubber component.

[41] The vulcanized rubber composition according to [39] or [40], wherein the rubber component contains a diene-based rubber.

[42] The vulcanized rubber composition according to [41], wherein the amount of the diene-based rubber in the rubber component is 50 to 100% by weight, more preferably 70 to 100% by weight, further preferably 80 to 100% by weight, and more preferably 100% by weight.

[43] The vulcanized rubber composition according to [39] or [40], wherein the rubber component contains a styrene-butadiene copolymer rubber.

[44] The vulcanized rubber composition according to [43], wherein the amount of the styrene-butadiene copolymer rubber in the rubber component is 50 to 100% by weight, more preferably 70 to 100% by weight, and further preferably 80 to 100% by weight.

[45] The vulcanized rubber composition according to [39] or [40], wherein the rubber component contains a styrene-butadiene copolymer rubber and a butadiene rubber.

[46] The vulcanized rubber composition according to [45], wherein the total amount of the styrene-butadiene copolymer rubber and butadiene rubber in the rubber component is 50 to 100% by weight, more preferably 70 to 100% by weight, further preferably 80 to 100% by weight, and most preferably 100% by weight.

[47] The vulcanized rubber composition according to [45] or [46], wherein the weight ratio of the amount of butadiene rubber to the amount of the styrene-butadiene copolymer rubber (the amount of the butadiene rubber/the amount of the styrene-butadiene copolymer rubber) is 5/95 to 50/50, more preferably 10/90 to 40/60, and further preferably 20/80 to 40/60.

[48] The vulcanized rubber composition according to [39] or [40], wherein the rubber component contains a styrene-butadiene copolymer rubber and a natural rubber.

[49] The vulcanized rubber composition according to [48], wherein the total amount of the styrene-butadiene copolymer rubber and natural rubber in the rubber component is 50 to 100% by weight, more preferably 70 to 100% by weight, further preferably 80 to 100% by weight, and most preferably 100% by weight.

[50] The vulcanized rubber composition according to [48] to [49], wherein the weight ratio of the amount of the natural rubber to the amount of the styrene-butadiene copolymer rubber (the amount of the natural rubber/the amount of the styrene-butadiene copolymer rubber) is 5/95 to 50/50, more preferably 10/90 to 40/60, and further preferably

20/80 to 40/60.

[51] The vulcanized rubber composition according to any one of [39] to [50], wherein the vulcanized rubber composition further contains silica.

[52] The vulcanized rubber composition according to [51], wherein the silica has a BET specific surface area of 20 to 400 $m^2/g$, more preferably 20 to 350 $m^2/g$, and further preferably 20 to 300 $m^2/g$.

[53] The vulcanized rubber composition according to [51] or [52], wherein the amount of the silica is 10 to 120 parts by weight, more preferably 20 to 120 parts by weight, further preferably 30 to 120 parts by weight, and most preferably 50 to 100 parts by weight per 100 parts by weight of the rubber component.

[54] The vulcanized rubber composition according to any one of [51] to [53], wherein the vulcanized rubber composition further contains carbon black.

[55] The vulcanized rubber composition according to [54], wherein the carbon black has a BET specific surface area of 10 to 130 $m^2/g$, more preferably 20 to 130 $m^2/g$, and further preferably 40 to 130 $m^2/g$.

[56] The vulcanized rubber composition according to [54] or [55], wherein the amount of the carbon black is 1 to 120 parts by weight, more preferably 1 to 100 parts by weight, further preferably 1 to 60 parts by weight, and most preferably 1 to 30 parts by weight per 100 parts by weight of the rubber component.

[57] The vulcanized rubber composition according to any one of [54] to [56], wherein the weight ratio of the amount of the carbon black to the amount of the silica (the amount of the carbon black/the amount of the silica) is 1/120 to 1/1, more preferably 1/120 to 3/5, further preferably 1/120 to 1/2, and most preferably 1/100 to 1/5.

[58] A tire comprising the vulcanized rubber composition according to any one of [1] to [57].

Advantageous Effect of Invention

According to the present invention, a vulcanized rubber composition excellent in abrasion resistance can be obtained.

Description of Embodiments

A vulcanized rubber composition containing a compound represented by the formula (I)(hereinafter, may be abbreviated as the "compound (I)") may be produced by kneading the compound (I), a rubber component and a sulfur component, and other components as required (e.g., silica, carbon black) and heating the resulting rubber composition. The compound (I) and the rubber component and/or other components are reacted to form another compound during the kneading or heating, and thus the compound (I) is considered to be consumed. For this reason, even when the compound (I) is used, the resulting vulcanized rubber composition may not contain the compound (I) in a predetermined amount. In this respect, as a result of intensive studies, the present inventors have found that a vulcanized rubber composition containing a predetermined amount of the compound (I) exhibits excellent abrasion resistance.

Hereinafter, the present invention will be described in sequence. Hereinafter, similarly to the "compound represented by the formula (I)", a "compound represented by the formula (II)" may be abbreviated as the "compound (II)". Compounds and the like represented by other formulas may be abbreviated in the same manner. Exemplification, preferable descriptions, and the like described below may be combined, unless these do not conflict with each other.

<Definitions>

First, definitions of substituents and the like used herein will be described in sequence.

"$C_{x-y}$" means that the number of carbon atoms is x or more and y or less (x and y each represent a number).

Examples of a halogen atom include a fluorine atom, a chlorine atom, a bromine atom, and an iodine atom.

An alkyl group may be either of linear and branched. The number of carbon atoms of the alkyl group is 1 to 18, for example. Examples of the alkyl group include a methyl group, an ethyl group, a propyl group, an isopropyl group, a butyl group, an isobutyl group, a sec-butyl group, a tert-butyl group, a pentyl group, an isopentyl group, a neopentyl group, a 1-ethylpropyl group, a hexyl group, an isohexyl group, a 1,1-dimethylbutyl group, a 2,2-dimethylbutyl group, a 3,3-dimethylbutyl group, a 2-ethylbutyl group, a heptyl group, an octyl group, a nonyl group, a decyl group, an undecyl group, a dodecyl group, a tridecyl group, a tetradecyl group, a pentadecyl group, a hexadecyl group, a heptadecyl group, and an octadecyl group.

The alkyl group optionally has a substituent. Other groups containing an alkyl group as a moiety (e.g. an alkoxy group) also optionally have a substituent. Examples of substituents that may be possessed by the alkyl groups (e.g., $C_{1-18}$ alkyl groups) and other groups containing an alkyl group (e.g., $C_{1-18}$ alkyl group) as a moiety include the following:

(1) a halogen atom,

(2) a cycloalkyl group (preferably a $C_{3-8}$ cycloalkyl group),

(3) an alkoxy group (preferably a $C_{1-6}$ alkoxy group),
(4) a cycloalkyloxy group (preferably a $C_{3-8}$ cycloalkyloxy group),
(5) an aryloxy group (preferably a $C_{6-14}$ aryloxy group),
(6) an aralkyloxy group (preferably a $C_{7-16}$ arakyloxy group), and
(7) an amino group optionally having a substituent.

The number of carbon atoms of the cycloalkyl group is 3 to 10, for example. Examples of the cycloalkyl group include a cyclopropyl group, a cyclobutyl group, a cyclopentyl group, a cyclohexyl group, a cycloheptyl group, a cyclooctyl group, a bicyclo[2.2.1]heptyl group, a bicyclo[2.2.2]octyl group, a bicyclo[3.2.1]octyl group, and an adamantyl group.
The number of carbon atoms of the aryl group is 6 to 18, for example. Examples of the aryl group include a phenyl group, a 1-naphthyl group, a 2-naphthyl group, a 1-anthryl group, a 2-anthryl group, and a 9-anthryl group.
The number of carbon atoms of the aralkyl group is 7 to 20, for example. Examples of the aralkyl group include a benzyl group, a phenethyl group, a naphthylmethyl group, and a phenylpropyl group.
The cycloalkyl group, aryl group, and aralkyl group each optionally have a substituent. Other groups containing a cycloalkyl group or the like as a moiety (e.g., cycloalkyloxy group or the like) also optionally have a substituent. Examples of substituents that may be possessed by the cycloalkyl group (e.g., $C_{3-10}$ cycloalkyl group), aryl group (e.g., $C_{6-18}$ aryl group), and aralkyl group (e.g., $C_{7-20}$ aralkyl group) and other groups containing such a group as a moiety include the following:

(1) a halogen atom,
(2) an alkyl group (preferably a $C_{1-6}$ alkyl group),
(3) a cycloalkyl group (preferably a $C_{3-8}$ cycloalkyl group),
(4) an aryl group (preferably a $C_{6-14}$ aryl group),
(5) an aralkyl group (preferably a $C_{7-16}$ aralkyl group),
(6) an alkoxy group (preferably a $C_{1-6}$ alkoxy group),
(7) a cycloalkyloxy group (preferably a $C_{3-8}$ cycloalkyloxy group),
(8) an aryloxy group (preferably a $C_{6-14}$ aryloxy group),
(9) an aralkyloxy group (preferably a $C_{7-16}$ arakyloxy group), and
(10) an amino group optionally having a substituent.

The description of an alkyl group as a moiety of an alkoxy group (i.e., alkyloxy group) is as described above. The same applies to the description of an alkyl group as a moiety of a group described below. Examples of the alkoxy group include a methoxy group, an ethoxy group, a propoxy group, an isopropoxy group, a butoxy group, an isobutoxy group, a sec-butoxy group, a tert-butoxy group, a pentyloxy group, and a hexyloxy group.
The description of a cycloalkyl group as a moiety of a cycloalkyloxy group is as described above. The same applies to the description of a cycloalkyl group as a moiety of a group described below. Examples of the cycloalkyloxy group include a cyclopropyloxy group, a cyclobutyloxy group, a cyclopentyloxy group, a cyclohexyloxy group, a cycloheptyloxy group, and a cyclooctyloxy group.
The description of an aryl group as a moiety of an aryloxy group is as described above. The same applies to the description of an aryl group as a moiety of a group described below. Examples of the aryloxy group include a phenyloxy group, a 1-naphthyloxy group, and a 2-naphthyloxy group.
The description of an aralkyl group as a moiety of an arakyloxy group is as described above. The same applies to the description of an aralkyl group as a moiety of a group described below. Examples of the arakyloxy group include a benzyloxy group, a phenethyloxy group, a naphthylmethyloxy group, and a phenylpropyloxy group.
Examples of the alkyl-carbonyloxy group include an acetyloxy group, a propanoyloxy group, a butanoyloxy group, a 2-methylpropanoyloxy group, a pentanoyloxy group, a 3-methylbutanoyloxy group, a 2-methylbutanoyloxy group, a 2,2-dimethylpropanoyloxy group, a hexanoyloxy group, and a heptanoyloxy group. A reference to a "$C_{1-18}$ alkyl-carbonyloxy group" means that the number of carbon atoms of the alkyl group as a moiety of this group is 1 to 18. Other references have the same meaning.
Examples of the cycloalkyl-carbonyloxy group include a cyclopropyl-carbonyloxy group, a cyclobutyl-carbonyloxy group, a cyclopentyl-carbonyloxy group, a cyclohexyl-carbonyloxy group, a cycloheptyl-carbonyloxy group, and a cyclooctylcarbonyloxy group.
Examples of the aryl-carbonyloxy group include a benzoyloxy group, a 1-naphthoyloxy group, and a 2-naphthoyloxy group.
Examples of the aralkyl-carbonyloxy group include a phenylacetyloxy group and a phenylpropionyloxy group.
Examples of the alkoxy-carbonyl group include a methoxycarbonyl group, an ethoxycarbonyl group, a propoxycarbonyl group, an isopropoxycarbonyl group, a butoxycarbonyl group, an isobutoxycarbonyl group, a secbutoxycarbonyl group, a tert-butoxycarbonyl group, a pentyloxycarbonyl group, and a hexyloxycarbonyl group.

Examples of the cycloalkyloxy-carbonyl group include a cyclopropyloxycarbonyl group, a cyclobutyloxycarbonyl group, a cyclopentyloxycarbonyl group, a cyclohexyloxycarbonyl group, a cycloheptyloxycarbonyl group, and a cyclooctyloxycarbonyl group.

Examples of the aryloxy-carbonyl group include a phenyloxycarbonyl group, a 1-naphthyloxycarbonyl group, and a 2-naphthyloxycarbonyl group.

Examples of the aralkyloxy-carbonyl group include a benzyloxycarbonyl group, a phenethyloxycarbonyl group, a naphthylmethyloxycarbonyl group, and a phenylpropyloxycarbonyl group.

Examples of the carbamoyl group optionally having a substituent include carbamoyl groups optionally having one or two substituents selected from an alkyl group optionally having a substituent, a cycloalkyl group optionally having a substituent, an aryl group optionally having a substituent, and an aralkyl group optionally having a substituent.

Preferred examples of the carbamoyl group optionally having a substituent include the following:

(1) a carbamoyl group,
(2) a mono- or di-(alkyl)carbamoyl group (the alkyl optionally has a substituent) (e.g., a methylcarbamoyl group, an ethylcarbamoyl group, a dimethylcarbamoyl group, a diethylcarbamoyl group, and a N-ethyl-N-methylcarbamoyl group),
(3) a mono- or di-(cycloalkyl)carbamoyl group (the cycloalkyl optionally has a substituent) (e.g., a cyclopropylcarbamoyl group and a cyclohexylcarbamoyl group),
(4) a mono- or di-(aryl)carbamoyl group (the aryl optionally has a substituent) (e.g., a phenylcarbamoyl group), and
(5) a mono- or di-(aralkyl)carbamoyl group (the aralkyl optionally has a substituent) (e.g., a benzylcarbamoyl group and a phenethylcarbamoyl group).

Here, the "mono- or di-(alkyl)carbamoyl group (the alkyl optionally has a substituent)" represents a mono(alkyl)carbamoyl group (the alkyl optionally has a substituent) or a di(alkyl)carbamoyl group (the alkyl optionally has a substituent). The "mono(alkyl)carbamoyl group (the alkyl optionally has a substituent)" represents a carbamoyl group having an alkyl group optionally having a substituent, and the "di(alkyl)carbamoyl group (the alkyl optionally has a substituent)" represents a carbamoyl group having two alkyl groups optionally having a substituent. The meaning of notations of mono- or di- is applicable to other groups.

Examples of the amino group optionally having a substituent include one or two amino groups optionally having a substituent selected from an alkyl group optionally having a substituent, a cycloalkyl group optionally having a substituent, an aryl group optionally having a substituent, an aralkyl group optionally having a substituent, an alkyl-carbonyl group optionally having a substituent, a cycloalkyl-carbonyl group optionally having a substituent, an aryl-carbonyl group optionally having a substituent, and an aralkyl-carbonyl group optionally having a substituent.

Preferred examples of the amino group optionally having a substituent include the following:

(1) an amino group,
(2) a mono- or di-(alkyl)amino group (the alkyl optionally has a substituent) (e.g., a methylamino group, a trifluoromethylamino group, a dimethylamino group, an ethylamino group, a diethylamino group, a propylamino group, and a dibutylamino group),
(3) a mono- or di-(cycloalkyl)amino group (the cycloalkyl optionally has a substituent) (e.g., a cyclopropylamino group and a cyclohexylamino group),
(4) a mono- or di-(aryl)amino group (the aryl optionally has a substituent) (e.g., a phenylamino group),
(5) a mono- or di-(aralkyl)amino group (the aralkyl optionally has a substituent) (e.g., a benzylamino group and a dibenzylamino group),
(6) a mono- or di-(alkyl-carbonyl)amino group (the alkyl-carbonyl optionally has a substituent) (e.g., an acetylamino group and a propionyl amino group),
(7) a mono- or di-(cycloalkyl-carbonyl)amino group (the cycloalkyl-carbonyl optionally has a substituent) (e.g., a cyclopropylcarbonylamino group and a cyclohexylcarbonylamino group),
(8) a mono- or di-(aryl-carbonyl)amino group (the aryl-carbonyl optionally has a substituent) (e.g., a benzoylamino group), and
(9) a mono- or di-(aralkyl-carbonyl)amino group (the aralkyl-carbonyl optionally has a substituent) (e.g., benzylcarbonylamino group).

Here, the "mono- or di-(alkyl)amino group (the alkyl optionally has a substituent)" represents a mono(alkyl)amino group (the alkyl optionally has a substituent) or a di(alkyl)amino group (the alkyl optionally has a substituent). The "mono(alkyl)amino group (the alkyl optionally has a substituent)" represents an amino group having an alkyl group optionally having a substituent, and the "di(alkyl)amino group (the alkyl optionally has a substituent)" represents an amino group two alkyl groups optionally having a substituent. The meaning of notations of mono- or di- is applicable to

other groups.

<Compound represented by the formula (I)>

One characteristic of the vulcanized rubber composition of the present invention is that the composition comprises a compound represented by the formula (I):

[Formula 5]

(I)

in a predetermined amount. One compound (I) may be used singly or two or more compounds (I) may be used in combination.

$X^{1a}$ represents a nitrogen atom or C-$R^{1a}$, $X^{3a}$ represents a nitrogen atom or C-$R^{3a}$, and $X^{5a}$ represents a nitrogen atom or C-$R^{5a}$, with the proviso that at least one of $X^{1a}$, $X^{3a}$, and $X^{5a}$ is a nitrogen atom. One or two of $X^{1a}$, $X^{3a}$, and $X^{5a}$ are preferably nitrogen atoms.

$R^{1a}$ and $R^{3a}$ to $R^{6a}$ each independently represent a hydrogen atom, a halogen atom, a $C_{1-18}$ alkyl group optionally having a substituent, a $C_{3-10}$ cycloalkyl group optionally having a substituent, a $C_{6-18}$ aryl group optionally having a substituent, a $C_{7-20}$ aralkyl group optionally having a substituent, a carboxy group, a $C_{1-18}$ alkoxy-carbonyl group optionally having a substituent, a $C_{3-10}$ cycloalkyloxy-carbonyl group optionally having a substituent, a $C_{6-18}$ aryloxy-carbonyl group optionally having a substituent, a $C_{7-20}$ aralkyloxy-carbonyl group optionally having a substituent, a carbamoyl group optionally having a substituent, a hydroxy group, a $C_{1-18}$ alkoxy group optionally having a substituent, a $C_{3-10}$ cycloalkyloxy group optionally having a substituent, a $C_{6-18}$ aryloxy group optionally having a substituent, a $C_{7-20}$ aralkyloxy group optionally having a substituent, a $C_{1-18}$ alkyl-carbonyloxy group optionally having a substituent, a $C_{3-10}$ cycloalkyl-carbonyloxy group optionally having a substituent, a $C_{6-18}$ aryl-carbonyloxy group optionally having a substituent, a $C_{7-20}$ aralkyl-carbonyloxy group optionally having a substituent, an amino group optionally having a substituent, or a nitro group. "$R^{3a}$ to $R^{6a}$" herein means "$R^{3a}$, $R^{4a}$, $R^{5a}$, and $R^{6a}$". Other similar references have the same meaning.

In one aspect of the present invention, $R^{1a}$ and $R^{3a}$ to $R^{6a}$ preferably each independently are a hydrogen atom, a $C_{1-18}$ alkyl group optionally having a substituent, a carboxy group, a $C_{1-18}$ alkoxy-carbonyl group optionally having a substituent, or a nitro group. In the present aspect, the $C_{1-18}$ alkyl group optionally having a substituent is preferably a $C_{1-12}$ alkyl group optionally having a halogen atom, more preferably a $C_{1-6}$ alkyl group optionally having a halogen atom, and further preferably a $C_{1-3}$ alkyl group optionally having a halogen atom. In the present aspect, the $C_{1-18}$ alkoxy-carbonyl group optionally having a substituent is preferably a $C_{1-12}$ alkoxy-carbonyl group optionally having a substituent and more preferably a $C_{1-12}$ alkoxy-carbonyl group.

Preferred examples of the compound (I) include the following:

(a) a compound in which both $X^{1a}$ and $X^{3a}$ are nitrogen atoms, $X^{5a}$ is C-$R^{5a}$, and $R^{4a}$ to $R^{6a}$ are each independently a hydrogen atom, a $C_{1-18}$ alkyl group optionally having a substituent, or a nitro group (hereinafter, may be abbreviated as the "compound (Ia)"),
(b) a compound in which $X^{1a}$ is a nitrogen atom, $X^{3a}$ is C-$R^{3a}$, $X^{5a}$ is C-$R^{5a}$, and $R^{3a}$ to $R^{6a}$ are each independently a hydrogen atom, a $C_{1-18}$ alkyl group optionally having a substituent, or a nitro group (hereinafter, may be abbreviated as the "compound (Ib)"), and
(c) a compound in which $X^{1a}$ is C-$R^{1a}$, $X^{3a}$ is C-$R^{3a}$, $X^{5a}$ is a nitrogen atom, and $R^{1a}$, $R^{3a}$, $R^{4a}$, and $R^{6a}$ are each independently a hydrogen atom, a $C_{1-18}$ alkyl group optionally having a substituent, or a nitro group (hereinafter, may be abbreviated as the "compound (Ic)").

In the compound (Ia), $R^{4a}$ to $R^{6a}$ are preferably each independently a hydrogen atom or a $C_{1-18}$ alkyl group optionally having a substituent. Among compounds (Ia), preferred is a compound in which both $X^{1a}$ and $X^{3a}$ are nitrogen atoms, $X^{5a}$ is C-$R^{5a}$, $R^{4a}$ and $R^{6a}$ are each independently a $C_{1-18}$ alkyl group optionally having a substituent, and $R^{5a}$ is a hydrogen atom.

In the compound (Ib), $R^{3a}$, $R^{4a}$, and $R^{6a}$ are preferably each independently a hydrogen atom or a $C_{1-18}$ alkyl

group optionally having a substituent, and are more preferably all hydrogen atoms. In the compound (Ib), $R^{5a}$ is preferably a hydrogen atom or a nitro group.

In the compound (Ic), $R^{1a}$, $R^{3a}$, $R^{4a}$, and $R^{6a}$ are preferably each independently a hydrogen atom or a $C_{1-18}$ alkyl group optionally having a substituent, and are more preferably all hydrogen atoms.

The $C_{1-18}$ alkyl group optionally having a substituent in the compound (Ia) to the compound (Ic) is preferably a $C_{1-18}$ alkyl group, more preferably a $C_{1-12}$ alkyl group, further preferably a $C_{1-6}$ alkyl group, and particularly preferably a $C_{1-3}$ alkyl group.

As for each of the compound (Ia) to the compound (Ic), one compound may be used singly or two or more compounds may be used in combination. Specific examples of the compound (Ia) to the compound (Ic) include the following. Among the following specific examples, the compound (Ia-1) is more preferred.

[Formula 6]

(Ia-1) (Ia-2) (Ib-1) (Ib-2) (Ic-1)

As the compound (I), commercially available products may be used. Examples of commercially available products include "4,6-dimethyl-2-mercaptopyrimidine" manufactured by Tokyo Chemical Industry Co., Ltd. (compound (Ia-1)), "2-mercaptopyrimidine" manufactured by Tokyo Chemical Industry Co., Ltd. (compound (Ia-2)), "2-mercaptopyridine" manufactured by Tokyo Chemical Industry Co., Ltd. (compound (Ib-1)), "2-mercapto-5-nitropyridine" manufactured by Tokyo Chemical Industry Co., Ltd. (compound (Ib-2)), and "4-mercaptopyridine" manufactured by Tokyo Chemical Industry Co., Ltd. (compound (Ic-1)). A compound obtained by introducing a substituent into a commercially available product by a known method also can be used as the compound (I).

The vulcanized rubber composition of the present invention is characterized by containing the compound (I) in a predetermined amount. The content of the compound (I) with respect to the entire vulcanized rubber composition is 0.00005% by weight or more, preferably 0.00008% by weight or more, more preferably 0.00010% by weight or more, further preferably 0.0010% by weight or more, and particularly preferably 0.010% by weight or more, from the viewpoint of abrasion resistance. Meanwhile, in order to produce a vulcanized rubber composition having a large content of the compound (I), it is necessary to increase the content of the compound (I) in an unvulcanized rubber composition for use in production of the vulcanized rubber composition. However, the processability of an unvulcanized rubber composition having a large content of the compound (I) is considered to be degraded because of scorch due to sulfanyl groups (-SH) of the compound (I). For this reason, the content of the compound (I) with respect to the entire vulcanized rubber composition is 5% by weight or less, preferably 3% by weight or less, and more preferably 1% by weight or less, from the viewpoint of prevention of degradation in the processability of the unvulcanized rubber composition. The content of the compound (I) in the present invention is a value obtained by extraction and analysis by the method and under the conditions described in the Example section.

<Compound represented by the formula (II)>

The vulcanized rubber composition of the present invention preferably contains a compound represented by the formula (II):

[Formula 7]

(II)

at a content of 0.0001 to 1.0% by weight with respect to the entire vulcanized rubber composition. A vulcanized rubber composition containing predetermined amounts of the compound (I) and the compound (II) can exert excellent abrasion resistance. One compound (II) may be used singly or two or more compounds (II) may be used in combination.

The vulcanized rubber composition containing the compound (I) and the compound (II) can be produced by kneading the compound (I), the compound (II), a rubber component and a sulfur component, and other components as required (e.g., silica, carbon black) and heating the resulting rubber composition. The compound (II) and the rubber component and/or other components are reacted and another compound is formed during the kneading or heating, and thus the compound (II), similarly to the compound (I), is considered to be consumed. For this reason, even when the compound (II) is used, the resulting vulcanized rubber composition may not contain the compound (II) in a predetermined amount.

$X^{1b}$ represents a nitrogen atom or C-$R^{1b}$, $X^{3b}$ represents a nitrogen atom or C-$R^{3b}$, $X^{5b}$ represents a nitrogen atom or C-$R^{5b}$, $X^{1c}$ represents a nitrogen atom or C-$R^{1c}$, $X^{3c}$ represents a nitrogen atom or C-$R^{3c}$, and $X^{5c}$ represents a nitrogen atom or C-$R^{5c}$, with the proviso that at least one of $X^{1b}$, $X^{3b}$, $X^{5b}$, $X^{1c}$, $X^{3c}$, and $X^{5c}$ is a nitrogen atom. One or more and five or less of $X^{1b}$, $X^{3b}$, $X^{5b}$, $X^{1c}$, $X^{3c}$, and $X^{5c}$ are preferably nitrogen atoms.

It is preferred that both $X^{1b}$ and $X^{1c}$ be nitrogen atoms or that $X^{1b}$ be C-$R^{1b}$ and $X^{1c}$ be C-$R^{1c}$. In the present aspect, $R^{1b}$ and $R^{1c}$ are preferably the same.

It is preferred that both $X^{3b}$ and $X^{3c}$ be nitrogen atoms or that $X^{3b}$ be C-$R^{3b}$ and $X^{3c}$ be C-$R^{3c}$. In the present aspect, $R^{3b}$ and $R^{3c}$ are preferably the same.

It is preferred that both $X^{5b}$ and $X^{5c}$ be nitrogen atoms or that $X^{5b}$ be C-$R^{5b}$ and $X^{5c}$ be C-$R^{5c}$. In the present aspect, $R^{5b}$ and $R^{5c}$ are preferably the same.

$R^{1b}$ and $R^{3b}$ to $R^{6b}$ and $R^{1c}$ and $R^{3c}$ to $R^{6c}$ each independently represent a hydrogen atom, a halogen atom, a $C_{1-18}$ alkyl group optionally having a substituent, a $C_{3-10}$ cycloalkyl group optionally having a substituent, a $C_{6-18}$ aryl group optionally having a substituent, a $C_{7-20}$ aralkyl group optionally having a substituent, a carboxy group, a $C_{1-18}$ alkoxy-carbonyl group optionally having a substituent, a $C_{3-10}$ cycloalkyloxy-carbonyl group optionally having a substituent, a $C_{6-18}$ aryloxy-carbonyl group optionally having a substituent, a $C_{7-20}$ aralkyloxy-carbonyl group optionally having a substituent, a carbamoyl group optionally having a substituent, a hydroxy group, a $C_{1-18}$ alkoxy group optionally having a substituent, a $C_{3-10}$ cycloalkyloxy group optionally having a substituent, a $C_{6-18}$ aryloxy group optionally having a substituent, a $C_{7-20}$ aralkyloxy group optionally having a substituent, a $C_{1-18}$ alkyl-carbonyloxy group optionally having a substituent, a $C_{3-10}$ cycloalkyl-carbonyloxy group optionally having a substituent, a $C_{6-18}$ aryl-carbonyloxy group optionally having a substituent, a $C_{7-20}$ aralkyl-carbonyloxy group optionally having a substituent, an amino group optionally having a substituent, or a nitro group.

In one aspect of the present invention, $R^{1b}$ and $R^{3b}$ to $R^{6b}$ and $R^{1c}$ and $R^{3c}$ to $R^{6c}$ are preferably each independently a hydrogen atom, a $C_{1-18}$ alkyl group optionally having a substituent, a carboxy group, a $C_{1-18}$ alkoxy-carbonyl group optionally having a substituent, or a nitro group. In the present aspect, the $C_{1-18}$ alkyl group optionally having a substituent is preferably a $C_{1-12}$ alkyl group optionally having a halogen atom, more preferably a $C_{1-6}$ alkyl group optionally having a halogen atom, and further preferably a $C_{1-3}$ alkyl group optionally having a halogen atom. In the present aspect, the $C_{1-18}$ alkoxy-carbonyl group optionally having a substituent is preferably a $C_{1-12}$ alkoxy-carbonyl group optionally having a substituent and more preferably a $C_{1-12}$ alkoxy-carbonyl group.

Preferred examples of the compound (II) include the following:

(a) a compound in which $X^{1b}$, $X^{3b}$, $X^{1c}$, and $X^{3c}$ are all nitrogen atoms, $X^{5b}$ is C-$R^{5b}$, $X^{5c}$ is C-$R^{5c}$, and $R^{4b}$ to $R^{6b}$ and $R^{4c}$ to $R^{6c}$ are each independently a hydrogen atom, a $C_{1-18}$ alkyl group optionally having a substituent, or a nitro group (hereinafter, may be abbreviated as the "compound (IIa)"),

(b) a compound in which both $X^{1b}$ and $X^{1c}$ are nitrogen atoms, $X^{3b}$ is C-$R^{3b}$, $X^{5b}$ is C-$R^{5b}$, $X^{3c}$ is C-$R^{3c}$, $X^{5c}$ is C-$R^{5c}$, and $R^{3b}$ to $R^{6b}$ and $R^{3c}$ to $R^{6c}$ are each independently a hydrogen atom, a $C_{1-18}$ alkyl group optionally having a substituent or a nitro group (hereinafter, may be abbreviated as the "compound (IIb)"), and

(c) a compound in which $X^{1b}$ is C-$R^{1b}$, $X^{3b}$ is C-$R^{3b}$, $X^{1c}$ is C-$R^{1c}$, $X^{3c}$ is C-$R^{3c}$, both $X^{5b}$ and $X^{5c}$ are nitrogen atoms, and $R^{1b}$,$R^{3b}$, $R^{4b}$, $R^{6b}$, $R^{1c}$, $R^{3c}$, $R^{4c}$, and $R^{6c}$ are each independently a hydrogen atom, a $C_{1-18}$ alkyl group optionally having a substituent, or a nitro group (hereinafter, may be abbreviated as the "compound (IIc)") .

In the compound (IIa) , $R^{4b}$ to $R^{6b}$ and $R^{4c}$ to $R^{6c}$ are preferably each independently a hydrogen atom and a $C_{1-18}$ alkyl group optionally having a substituent. Among compounds (IIa), preferred is a compound in which $X^{1b}$, $X^{3b}$, $X^{1c}$, and $X^{3c}$ are all nitrogen atoms, $X^{5b}$ is C-$R^{5b}$, $X^{5c}$ is C-$R^{5c}$, $R^{4b}$, $R^{6b}$, $R^{4c}$, and $R^{6c}$ are each independently a $C_{1-18}$ alkyl group optionally having a substituent, and both $R^{5b}$ and $R^{5c}$ are hydrogen atoms.

In the compound (IIb) , $R^{3b}$, $R^{4b}$, $R^{6b}$, $R^{3c}$, $R^{4c}$, and $R^{6c}$ are preferably each independently a hydrogen atom or a $C_{1-18}$ alkyl group optionally having a substituent and more preferably all hydrogen atoms. In the compound (IIb), $R^{5b}$ and $R^{5c}$ are preferably a hydrogen atom or a nitro group.

In the compound (IIc) , $R^{1b}$, $R^{3b}$, $R^{4b}$, $R^{6b}$, $R^{1c}$, $R^{3c}$, $R^{4c}$, and $R^{6c}$ are preferably each independently a hydrogen

atom or a $C_{1-18}$ alkyl group optionally having a substituent and more preferably all hydrogen atoms.

The $C_{1-18}$ alkyl group optionally having a substituent in the compound (IIa) to the compound (IIc) is preferably a $C_{1-18}$ alkyl group, more preferably a $C_{1-12}$ alkyl group, further preferably a $C_{1-6}$ alkyl group, and particularly preferably a $C_{1-3}$ alkyl group.

As for each of the compound (IIa) to the compound (IIc), one compound may be used singly or two or more compounds may be used in combination. Specific examples of the compound (IIa) to the compound (IIc) include the following. Among the following specific examples, the compound (IIa-1) is more preferred.

[Formula 8]

(IIa-1) (IIa-2) (IIb-1)

(IIb-2) (IIc-1)

As the compound (II), commercially available products may be used. Examples of commercially available products include "2,2'-dipyridyl disulfide" manufactured by Tokyo Chemical Industry Co., Ltd. (compound (IIb-1)), "2,2'-dithiobis(5-nitropyridine)" manufactured by Tokyo Chemical Industry Co., Ltd. (compound (IIb-2)), and "4,4'-dipyridyl disulfide" manufactured by Tokyo Chemical Industry Co., Ltd. (compound (IIc-1)) .

The compound (II) can be produced in accordance with known methods. The compound (II) may be produced by, for example, as shown below, oxidization and formation of a disulfide bond of a commercially available compound (I) and/or a compound (I) produced by a known method (the groups in the following formula are as described above).

[Formula 9]

(Id) (Ie) (II)

Oxidization in the synthesis reaction of the compound (II) may be conducted by using an oxidizing agent such as hydrogen peroxide, potassium ferricyanide, oxygen, iodine, bromine, iodobenzene diacetate, sodium periodate, or potassium permanganate. One alone oxidizing agent or two or more oxidizing agents may be used either singly or as combined. Alternatively, hydrogen peroxide and sodium iodide may be combined to generate iodine in the system. The amount of the oxidizing agent to be used (when two or more oxidizing agents are used, the total amount thereof) is preferably 1 to 10 mol and more preferably 1 to 3 mol with respect to 1 mol in total of the compound (Id) and the compound (Ie).

The synthesis reaction of the compound (II) (i.e., oxidization and formation of a disulfide bond) is conducted usually in a solvent. Examples of this solvent include esterbased solvents such as ethyl acetate, methyl acetate, butyl acetate, propyl acetate, isopropyl acetate, and ethyl lactate, amide-based solvents such as N,N-dimethylformamide, N,N-dimethylacetamide, and N-methyl-2-pyrrolidone, sulfoxide-based solvents such as dimethylsulfoxide, aromatic hydrocarbon solvents such as benzene, toluene, and xylene, ether-based solvents such as tetrahydrofuran, 1,4-dioxane, and methyl ethyl ether, and protic solvents such as water, methanol, and ethanol. One alone solvent or two or more solvents may be used either singly or as combined.

The synthesis reaction of the compound (II) (i.e., oxidization and formation of a disulfide bond) is preferably conducted by adding a hydrogen peroxide aqueous solution to the compound (Id) and the compound (Ie). The oxidization and formation of a disulfide bond by use of a hydrogen peroxide aqueous solution is an exothermic reaction. After addition of the hydrogen peroxide aqueous solution, the mixture is preferably stirred at 0 to 100°C and more preferably 0 to 60°C for preferably 0.1 to 48 hours and more preferably 0.1 to 24 hours.

After the synthesis of the compound (II), the compound (II) can be obtained by a known means (filtration, extraction, concentration, or the like). The resulting compound (II) may be purified by a known means.

The content of the compound (II) with respect to the entire vulcanized rubber composition is preferably 0.0001% by weight or more, more preferably 0.0002% by weight or more, further preferably 0.0005% by weight or more, and particularly preferably 0.0010% by weight or more, from the viewpoint of abrasion resistance. Meanwhile, the content is preferably 1.0% by weight or less, more preferably 0.5% by weight or less, further preferably 0.3% by weight or less, particularly preferably 0.2% by weight or less, and most preferably 0.1% by weight or less. The content of the compound (II) in the present invention is a value obtained by extraction and analysis by the method and under the conditions described in the Example section.

<Vulcanized rubber>

The vulcanized rubber composition of the present invention contains a vulcanized rubber. The "vulcanized rubber" here means a "rubber component crosslinked with a sulfur component". Hereinafter, the rubber component and the sulfur component will be described in sequence.

(Rubber component)

Examples of the rubber component include a styrene-butadiene copolymer rubber (SBR), a natural rubber (NR) (modified natural rubber including, for example, epoxidized natural rubber and deproteinized natural rubber), a butadiene rubber (BR), an isoprene rubber (IR), a nitrile rubber (NBR), a chloroprene rubber (CR), a butyl rubber (isoprene-isobutyrene copolymer rubber, IIR), an ethylene-propylene-diene copolymer rubber (EPDM), and a halogenated butyl rubber (HR). One alone rubber component or two or more rubber components may be used either singly or as combined.

Examples of the SBR include emulsion-polymerized SBRs and solution-polymerized SBRs described on pages 210 to 211 in Gomu Kogyo Binran (Rubber Industry Handbook), 4th edition edited by Society of Rubber Industry, Japan. An emulsion-polymer SBR and a solution-polymerized SBR may be used in combination.

Examples of the solution-polymerized SBR include modified solution-polymerized SBRs having at least one element of nitrogen, tin, and silicone at a molecular end, obtained by modification with a modifier. Examples of the modifier include lactam compounds, amide compounds, urea compounds, N,N-dialkylacrylamide compounds, isocyanate compounds, imide compounds, silane compounds having an alkoxy group, aminosilane compounds, combined modifiers of a tin compound and a silane compound having an alkoxy group, and combined modifiers of an alkylacrylamide compound and a silane compound having an alkoxy group. These modifiers may be used alone, or a plurality of these may be used. Specific examples of the modified solution-polymerized SBR include solution-polymerized SBRs obtained by modifying a molecular end using 4,4'-bis(dialkylamino)benzophenone such as "Nipol(R) NS116" manufactured by Nippon Zeon Co., Ltd., solution-polymerized SBRs obtained by modifying a molecular end using a halogenated tin compound such as "SL574" manufactured by JSR Corporation, and silane-modified solution-polymerized SBRs such as "E10" and "E15" manufactured by Asahi Kasei Corporation.

Also can be used are oil-extended SBRs obtained by adding an oil such as a process oil or an aroma oil to emulsion-polymerized SBRs and solution-polymerized SBRs.

Examples of the natural rubber include natural rubbers of RSS#1, RSS#3, TSR20, SIR20 grades or the like. Examples of epoxidized natural rubbers include those having a degree of epoxidation of 10 to 60 mol% (e.g., ENR25 and ENR50 manufactured by Kumpulan Guthrie Bhd.). Examples of deproteinized natural rubbers include deproteinized natural rubbers having a content of total nitrogen of 0.3% by weight or less. Examples of other modified natural rubbers include modified natural rubbers having a polar group obtained by reacting 4-vinylpyridine, N,N,-dialkylaminoethyl acrylate (e.g., N,N,-diethylaminoethyl acrylate), 2-hydroxy acrylate, or the like with a natural rubber.

As the BR, BRs that are common in the tire industry can be used. The BR is often used as a blend of a SBR and/or a natural rubber.

As the BR, BRs having a high cis content are preferred because of being highly effective for improving the abrasion resistance, and high-cis BRs having a high-cis content of 95% by mass or more are more preferred. Examples of the high-cis BR include BR1220 manufactured by Nippon Zeon Co., Ltd. and BR150B manufactured by Ube Industries, Ltd.

It is also possible to use a modified BR having at least one element of nitrogen, tin, and silicone at a molecular end, obtained by modification with a modifier. Examples of the modifier include 4,4'-bis(dialkylamino)benzophenone,

halogenated tin compounds, lactam compounds, amide compounds, urea compounds, N,N-dialkylacrylamide compounds, isocyanate compounds, imide compounds, silane compounds having an alkoxy group (e.g., a trialkoxysilane compound), aminosilane compounds, tin compounds, and alkylacrylamide compounds. These modifiers may be used alone, or a plurality of these may be used. Examples of the modified BR include tin-modified BRs such as "Nipol(R) BR1250H" manufactured by Nippon Zeon Co., Ltd.

The rubber component preferably contains a diene-based rubber. Here, the diene-based rubber means a rubber produced from a diene monomer having a conjugated double bond as a raw material. Examples of the diene-based rubber include a styrene-butadiene copolymer rubber (SBR), a natural rubber (NR), a butadiene rubber (BR), an isoprene rubber (IR), a nitrile rubber (NBR), and a chloroprene rubber.

When a diene-based rubber is used, the amount of the diene-based rubber in the rubber component (i.e., the amount of the diene-based rubber per 100% by weight of the rubber component) is preferably 50 to 100% by weight, more preferably 70 to 100% by weight, further preferably 80 to 100% by weight, and most preferably 100% by weight. That is, the rubber component is most preferably constituted by a diene-based rubber.

In one aspect of the present invention, the rubber component preferably contains an SBR. The amount of the SBR in the rubber component in the present aspect is preferably 50 to 100% by weight, more preferably 70 to 100% by weight, and further preferably 80 to 100% by weight.

In one aspect of the present invention, the rubber component preferably contains a SBR and a BR. In the present aspect, the total amount of the SBR and BR in the rubber component is preferably 50 to 100% by weight, more preferably 70 to 100% by weight, further preferably 80 to 100% by weight, and most preferably 100% by weight. That is, in the present aspect, the rubber component is most preferably constituted by a SBR and a BR. In the present aspect, the weight ratio of the amount of the BR to the amount of the SBR (the amount of the BR/the amount of the SBR) is preferably 5/95 to 50/50, more preferably 10/90 to 40/60, and further preferably 20/80 to 40/60, from the viewpoint of fuel consumption efficiency and abrasion resistance.

In one aspect of the present invention, the rubber component preferably contains a SBR and a natural rubber. In the present aspect, the total amount of the SBR and natural rubber in the rubber component is preferably 50 to 100% by weight, more preferably 70 to 100% by weight, further preferably 80 to 100% by weight, and most preferably 100% by weight. That is, in the present aspect, the rubber component is most preferably constituted by a SBR and a natural rubber. In the present aspect, the weight ratio of the amount of the natural rubber to the amount of the SBR (the amount of the natural rubber/the amount of the SBR) is preferably 5/95 to 50/50, more preferably 10/90 to 40/60, and further preferably 20/80 to 40/60, from the viewpoint of improvement in durability.

(Sulfur component)

Examples of the sulfur component include powder sulfur, precipitated sulfur, colloidal sulfur, insoluble sulfur, and highly dispersible sulfur.

The amount of the sulfur component is preferably 0.1 to 10 parts by weight, more preferably 0.1 to 7 parts by weight, and further preferably 0.1 to 4 parts by weight per 100 parts by weight of the rubber component.

<Other components>

In the present invention, other components may be used, which are different from the compound (I), compound (II), and vulcanized rubber (i.e., rubber component crosslinked by a sulfur component) described above. As the other components, components known in the field of rubber can be used, and examples of the components include a filler, a compound that can be bonded to silica (e.g., silane coupling agent), a vulcanization accelerator, a vulcanization accelerating aid, an anti-aging agent, a processing aid, an oil, a wax, resorcinol, a resin, a viscoelasticity improving agent, a peptizing agent, a retarder, a compound having oxyethylene units, and a catalyst (cobalt naphthenate and the like). One of each of the other components or two or more of each of the other components may be used either singly or as combined.

(Filler)

Examples of the filler include silica, carbon black, aluminum hydroxide, pulverized bituminous coal, talc, clay (particularly, calcined clay), and titanium oxide. One alone filler or two or more fillers may be used either singly or as combined.

Examples of the silica include (i) silica having a pH of 6 to 8, (ii) silica containing 0.2 to 1.5% by weight of sodium, (iii) perfectly spherical silica having a circularity of 1 to 1.3, (iv) silica surface-treated with a silicone oil (e.g., dimethylsilicone oil), an organosilicon compound containing an ethoxysilyl group, an alcohol (e.g., ethanol, polyethylene glycol), or the like, and (v) mixtures of two or more silicas each having a different surface area. One alone silica or two or more silicas may be used either singly or as combined.

Silica has a BET specific surface area of preferably 20 to 400 $m^2$/g, more preferably 20 to 350 $m^2$/g, and further preferably 20 to 300 $m^2$/g. The BET specific surface area can be measured by a multipoint nitrogen adsorption method (BET method).

Examples of commercially available silica products include "Nipsil(R) AQ" and "Nipsil(R) AQ-N" manufactured by Tosoh Silica Corporation, "Ultrasil(R) VN3", "Ultrasil(R) VN3-G", "Ultrasil(R) 360", "Ultrasil(R) 7000", and "Ultrasil(R) 9100GR" manufactured by Evonik Industries AG, and "Zeosil(R) 115GR", "Zeosil(R) 1115MP", "Zeosil(R) 1205MP", and "Zeosil(R) Z85MP" manufactured by Solvay S.A.

When silica is used, the amount of the silica is preferably 10 to 120 parts by weight, more preferably 20 to 120 parts by weight, further preferably 30 to 120 parts by weight, and most preferably 50 to 100 parts by weight per 100 parts by weight of the rubber component, from the viewpoint of abrasion resistance.

Examples of the carbon black include carbon blacks described on page 494 in Gomu Kogyo Binran (Rubber Industry Handbook), 4th edition edited by Society of Rubber Industry, Japan. One alone carbon black or two or more carbon blacks may be used either singly or as combined. As the carbon black, HAF (High Abrasion Furnace), SAF (Super Abrasion Furnace), ISAF (Intermediate SAF), ISAF-HM (Intermediate SAF-High Modulus), FEF (Fast Extrusion Furnace), MAF (Medium Abrasion Furnace), GPF (General Purpose Furnace), and SRF (Semi-Reinforcing Furnace) are preferred.

The carbon black has a BET specific surface area of preferably 10 to 130 $m^2$/g, more preferably 20 to 130 $m^2$/g, and further preferably 40 to 130 $m^2$/g. The BET specific surface area can be measured by a multipoint nitrogen adsorption method (BET method).

When a carbon black is used, the amount of the carbon black is preferably 1 to 120 parts by weight, more preferably 1 to 100 parts by weight, further preferably 1 to 60 parts by weight, and most preferably 1 to 30 parts by weight per 100 parts by weight of the rubber component, from the viewpoint of abrasion resistance.

When carbon black is used, the weight ratio of the amount of the carbon black to the amount of the silica (the amount of the carbon black/the amount of the silica) is preferably 1/120 to 1/1, more preferably 1/120 to 3/5, further preferably 1/120 to 1/2, and most preferably 1/100 to 1/5, from the viewpoint of abrasion resistance.

Examples of aluminum hydroxide include aluminum hydroxides having a nitrogen adsorption specific surface area of 5 to 250 $m^2$/g and a DOP oiling quantity of 50 to 100 ml/100 g.

The average particle size of the pulverized bituminous coal is preferably 0.001 mm or more, preferably 0.1 mm or less, more preferably 0.05 mm or less, and further preferably 0.01 mm or less. The average particle size of the pulverized bituminous coal is an average particle size on a mass basis as calculated from a particle size distribution measured in accordance with JIS Z 8815-1994.

The specific gravity of the pulverized bituminous coal is preferably 1.6 or less, more preferably 1.5 or less, and further preferably 1.3 or less. When a pulverized bituminous coal having a specific gravity of more than 1.6 is used, the specific gravity of the entire rubber composition increases, and the fuel consumption efficiency of a tire may not be sufficiently improved. The specific gravity of the pulverized bituminous coal is preferably 0.5 or more and more preferably 1.0 or more. When a pulverized bituminous coal having a specific gravity of less than 0.5 is used, processability on kneading may be degraded.

(Compound that can be bonded to silica)

Examples of the compound that can be bonded to silica include bis(3-triethoxysilylpropyl)tetrasulfide (e.g., "Si-69" manufactured by Evonik Industries AG), bis(3-triethoxysilylpropyl)disulfide (e.g., "Si-75" manufactured by Evonik Industries AG), bis(3-diethoxymethylsilylpropyl)tetrasulfide, bis(3-diethoxymethylsilylpropyl)disulfide, 3-octanoylthiopropyltriethoxysilane (alias: "octanethioic acid S-[3-(triethoxysilyl)propyl]ester", e.g., "NXT Silane" manufactured by General Electric Silicones), octanethioic acid S-[3-{(2-methyl-1,3-propanedialkoxy)ethoxysilyl}propyl]ester, octanethioic acid S-[3-{(2-methyl-1,3-propanedialkoxy)methylsilyl}propyl] ester, methyltrimethoxysilane, methyltriethoxysilane, methyltriacetoxysilane, methyltributoxysilane, ethyltrimethoxysilane, ethyltriethoxysilane, isobutyltrimethoxysilane, isobutyltriethoxysilane, n-octyltrimethoxysilane, n-octyltriethoxysilane, vinyltrimethoxysilane, vinyltriethoxysilane, vinyltris(methoxyethoxy)silane, phenyltrimethoxysilane, phenyltriethoxysilane, phenyltriacetoxysilane, 3-methacryloxypropyltrimethoxysilane, 3-methacryloxypropyltriethoxysilane, 3-aminopropyltrimethoxysilane, 3-aminopropyltriethoxysilane, N-(2-aminoethyl)-3-aminopropyltrimethoxysilane, N-(2-aminoethyl)-3-aminopropyltriethoxysilane, (3-glycidoxypropyl)trimethoxysilane, (3-glycidoxypropyl)triethoxysilane, 2-(3,4-epoxycyclohexyl)ethyltrimethoxysilane, 2-(3,4-epoxycyclohexyl)ethyltriethoxysilane, 3-isocyanatopropyltrimethoxysilane, and 3-isocyanatopropyltriethoxysilane. Among these, bis(3-triethoxysilylpropyl)tetrasulfide (e.g., "Si-69" manufactured by Evonik Industries AG), bis(3-triethoxysilylpropyl)disulfide (e.g., "Si-75" manufactured by Evonik Industries AG), and 3-octanoylthiopropyltriethoxysilane (e.g., "NXT Silane" manufactured by General Electric Silicones) are preferred.

When a compound that can be bonded to silica is used, the amount of the compound is preferably 2 to 20 parts by weight, more preferably 2 to 15 parts by weight, and further preferably 2 to 10 parts by weight per 100 parts by weight

of the silica.

When a compound that can be bonded to silica is used, a monohydric alcohol such as ethanol, butanol, and octanol; a polyhydric alcohol such as ethylene glycol, diethylene glycol, triethylene glycol, polyethylene glycol, polypropylene glycol, pentaerythritol, and polyether polyol; a N-alkylamine; an amino acid; or a liquid polybutadiene having a carboxy-modified or amine-modified molecular end may be used.

(Vulcanization accelerator)

As the vulcanization accelerator, for example, those described in Gomu Kogyo Binran (Rubber Industry Handbook), 4th edition (published by The Society of Rubber Science and Technology, Japan, on Jan. 20, 1994) may be used. One alone vulcanization accelerator or two or more vulcanization accelerators may be used either singly or as combined. Examples of the vulcanization accelerator include sulfenamide-based vulcanization accelerators, thiazole-based vulcanization accelerators, and guanidine-based vulcanization accelerators.

Examples of the sulfenamide-based vulcanization accelerator include N-cyclohexyl-2-benzothiazolylsulfenamide (CBS), N-tert-butyl-2-benzothiazolylsulfenamide (BBS), N-oxydiethylene-2-benzothiazolylsulfenamide (OBS), and N,N-dicyclohexyl-2-benzothiazolylsulfenamide (DCBS). One alone sulfenamide-based vulcanization accelerator or two or more sulfenamide vulcanization accelerators may be used either singly or as combined.

Examples of the thiazole-based vulcanization accelerator include 2-mercaptobenzothiazole (MBT), dibenzothiazolyl disulfide (MBTS), 2-mercaptobenzothiazole cyclohexylamine salts (CMBT), and 2-mercaptobenzothiazole zinc salts (ZMBT). One alone thiazole-based vulcanization accelerator or two or more thiazole vulcanization accelerators may be used either singly or as combined.

Examples of the guanidine-based vulcanization accelerator include diphenylguanidine (DPG) and N,N'-di-o-tolyl guanidine (DOTG). One alone guanidine-based vulcanization accelerator or two or more guanidine vulcanization accelerators may be used either singly or as combined.

When a vulcanization accelerator is used, the amount of the vulcanization accelerator (when two or more vulcanization accelerators are used, the total amount thereof) is preferably 0.5 to 10.5 parts by weight, more preferably 0.7 to 8 parts by weight, and further preferably 0.8 to 5.5 parts by weight per 100 parts by weight of the rubber component.

The weight ratio of the amount of the sulfur component to the amount of the vulcanization accelerator (the amount of the sulfur component/the amount of the vulcanization accelerator) is not particularly limited and is preferably 1/10 to 10/1 and more preferably 1/5 to 5/1. When two or more vulcanization accelerators (e.g., CBS and DPG) are used, the weight ratio is calculated by using the amount of the sulfur component and the total amount of the two or more vulcanization accelerators.

(Vulcanization accelerating aid)

Examples of the vulcanization accelerating aid include zinc oxide, citraconimide compounds, alkylphenol-sulfur chloride condensates, organic thiosulfate compounds, and compounds represented by the formula (III):

$$R^{16}\text{-S-S-}R^{17}\text{-S-S-}R^{18} \qquad \text{(III)}$$

(wherein, $R^{17}$ represents a $C_{2-10}$ alkanediyl group, and $R^{16}$ and $R^{18}$ each independently represent a monovalent organic group containing a nitrogen atom.).

In the present invention, zinc oxide is encompassed by the concept of the vulcanization accelerating aid and is not encompassed by the concept of the filler described above.

When zinc oxide is used, the amount of the zinc oxide is preferably 0.01 to 20 parts by weight, more preferably 0.1 to 15 parts by weight, and further preferably 0.1 to 10 parts by weight per 100 parts by weight of the rubber component.

As the citraconimide compound, biscitraconimides are preferred because of being thermally stable and excellent in dispersibility into a rubber component. Specific examples thereof include 1,2-biscitraconimide methylbenzene, 1,3-biscitraconimide methylbenzene, 1,4-biscitraconimide methylbenzene, 1,6-biscitraconimide methylbenzene, 2,3-biscitraconimide methyltoluene, 2,4-biscitraconimide methyltoluene, 2,5-biscitraconimide methyltoluene, 2,6-biscitraconimide methyltoluene, 1,2-biscitraconimide ethylbenzene, 1,3-biscitraconimide ethylbenzene, 1,4-biscitraconimide ethylbenzene, 1,6-biscitraconimide ethylbenzene, 2,3-biscitraconimide ethyltoluene, 2,4-biscitraconimide ethyltoluene, 2,5-biscitraconimide ethyltoluene, and 2,6-biscitraconimide ethyltoluene.

Among citraconimide compounds, 1,3-biscitraconimide methylbenzene represented by the following formula is preferred because of being particularly thermally stable, particularly excellent in dispersibility into a rubber component, and enabling a vulcanized rubber composition having a high hardness (Hs) to be obtained (reversion suppression).

[Formula 10]

As the vulcanization accelerating aid, because of enabling a vulcanized rubber composition having a high hardness (Hs) to be obtained, an alkylphenol-sulfur chloride condensate represented by the formula (IV):

[Formula 11]

(IV)

[wherein, n is an integer of 0 to 10, X is an integer of 2 to 4, and $R^{19}$ is a $C_{5-12}$ alkyl group.] is preferably used.

n in the formula (IV) is preferably an integer of 1 to 9 because the dispersibility of the alkylphenol-sulfur chloride condensate (IV) into a rubber component is good.

When X exceeds 4, the alkylphenol-sulfur chloride condensate (IV) tends to be thermally unstable. When X is 1, the sulfur content (the weight of sulfur) in the alkylphenol-sulfur chloride condensate (IV) is low. X is preferably 2 because a high hardness can be efficiently developed (reversion suppression).

$R^{19}$ is a $C_{5-12}$ alkyl group. $R^{19}$ is preferably a $C_{6-9}$ alkyl group because the dispersibility of the alkylphenol-sulfur chloride condensate (IV) into a rubber component is good.

A specific example of the alkylphenol-sulfur chloride condensate (IV) is TACKIROL V200 manufactured by Taoka Chemical Co., Ltd., in which, in the formula (IV), n is 0 to 10, X is 2, and $R^{19}$ is an octyl group and which has a sulfur content of 24% by weight.

As the vulcanization accelerating aid, from the viewpoint that a vulcanized rubber composition having a high hardness (Hs) can be obtained (reversion suppression), a salt of organic thiosulfate compound (hereinafter, may be denoted by the "organic thiosulfate compound salt (V)") represented by the formula (V):

$$HO_3S\text{-}S\text{-}(CH_2)\,s\text{-}S\text{-}SO_3H \qquad (V)$$

[wherein, s is an integer of 3 to 10.] is preferably used. An organic thiosulfate compound salt (V) containing crystalline water may be used. Preferable examples of the organic thiosulfate compound salt (V) include lithium salts, potassium salts, sodium salts, magnesium salts, calcium salts, barium salts, zinc salts, nickel salts, and cobalt salt, and potassium salts and sodium salts are preferred.

s is an integer of 3 to 10 and preferably an integer of 3 to 6. When s is 2 or less, no sufficient thermal fatigue resistance tends to be obtained. When s is 11 or more, no sufficient effect of improving thermal fatigue resistance by the organic thiosulfate compound salt (V) may be obtained.

As the organic thiosulfate compound salt (V), from the viewpoint of being stable under normal temperature and pressure, a sodium salt monohydrate and a sodium salt dihydrate thereof are preferred. From the viewpoint of costs, an organic thiosulfate compound salt (V) obtained from sodium thiosulfate is more preferred, and sodium 1,6-hexamethylene dithiosulfate dihydrate represented by the following formula is further preferred.

[Formula 12]

$$^{+}Na^{-}O_3S-S-(CH_2)_6-S-SO_3^{-}Na^{+}\cdot 2H_2O$$

Because of being dispersed well into the rubber component and, when used in combination with an alkylphenol-sulfur chloride condensate (IV), being inserted at the midpoint of the -Sx-crosslinkage of the alkylphenol-sulfur chloride condensate (IV) to enable a hybrid crosslinkage with the alkylphenol-sulfur chloride condensate (IV) to be formed, a compound represented by the formula (III):

$$R^{16}\text{-S-S-}R^{17}\text{-S-S-}R^{18} \qquad \text{(III)}$$

(wherein, $R^{17}$ represents a $C_{2-10}$ alkanediyl group, and $R^{16}$ and $R^{18}$ each independently represent a monovalent organic group containing a nitrogen atom.) is preferably used as the vulcanization accelerating aid.

$R^{17}$ is a $C_{2-10}$ alkanediyl group, preferably a $C_{4-8}$ alkanediyl group, and more preferably a linear $C_{4-8}$ alkanediyl group. $R^{17}$ is preferably linear. When $R^{17}$ has one or less carbon atom, thermal stability may be poor. When $R^{17}$ has 11 or more carbon atoms, the distance between polymers via the vulcanization accelerating aid becomes longer, and the effect of addition of the vulcanization accelerating aid may not be obtained.

$R^{16}$ and $R^{18}$ are each independently a monovalent organic group containing a nitrogen atom. As the monovalent organic group containing a nitrogen atom, monovalent organic groups containing at least one aromatic ring are preferred, and monovalent organic groups containing an aromatic ring and a =N-C(=S)- group are more preferred. $R^{16}$ and $R^{18}$ each may be the same or different, but are preferably the same for the reasons such as ease of production.

Examples of the compound (III) include 1,2-bis(dibenzylthiocarbamoyldithio)ethane, 1,3-bis(dibenzylthiocarbamoyldithio)propane, 1,4-bis(dibenzylthiocarbamoyldithio)butane, 1,5-bis(dibenzylthiocarbamoyldithio)pentane, 1,6-bis(dibenzylthiocarbamoyldithio)hexane, 1,7-bis(dibenzylthiocarbamoyldithio)heptane, 1,8-bis(dibenzylthiocarbamoyldithio)octane, 1,9-bis(dibenzylthiocarbamoyldithio)nonane, and 1,10-bis(dibenzylthiocarbamoyldithio)decane. Among these, 1,6-bis(dibenzylthiocarbamoyldithio)hexane is preferred because of being thermally stable and excellent in dispersibility into a rubber component.

Examples of a commercially available product of the compound (III) include VULCUREN TRIAL PRODUCT KA9188 and VULCUREN VPKA9188 (1,6-bis(dibenzylthiocarbamoyldithio)hexane) manufactured by Bayer AG.

(Anti-aging agent)

Examples of the anti-aging agent include those described on pages 436 to 443 in Gomu Kogyo Binran (Rubber Industry Handbook), 4th edition edited by Society of Rubber Industry, Japan. As the anti-aging agent, N-phenyl-N'-1,3-dimethylbutyl-p-phenylenediamine (abbreviation "6PPD", for example, "Antigen(R) 6C" manufactured by SUMITOMO CHEMICAL COMPANY, LIMITED), a reaction product of aniline and acetone (abbreviation "TMDQ"), poly(2,2,4-trimethyl-1,2-)dihydroquinoline) (e.g., Antioxidant FR manufactured by MATSUBARA INDUSTRIES, INC.), synthetic waxes (paraffin wax and the like), and vegetable waxes are preferably used.

When an anti-aging agent is used, the amount of the anti-aging agent is preferably 0.01 to 15 parts by weight, more preferably 0.1 to 10 parts by weight, and further preferably 0.1 to 5 parts by weight per 100 parts by weight of the rubber component.

(Processing aid)

Examples of the processing aid include fatty acids such as ricinoleic acid, palmitic acid, stearic acid, and oleic acid, amides and esters thereof, and fatty acid metal salts such as zinc stearate, barium stearate, calcium stearate, and zinc laurate. Examples of commercially available products include "STRUKTOL A50P", "STRUKTOL A60", "STRUKTOL EF44", "STRUKTOL HT204", "STRUKTOL HT207", "STRUKTOL HT254", "STRUKTOL HT266", and "STRUKTOL WB16" manufactured by SCHILL & SEILACHER Gmbh. & CO.

When a processing aid is used, the amount of the processing aid is preferably 0.01 to 20 parts by weight, more preferably 0.1 to 15 parts by weight, and further preferably 0.1 to 10 parts by weight per 100 parts by weight of the rubber component.

When stearic acid is used as the processing aid, the amount of stearic acid is preferably 0.01 to 15 parts by weight, more preferably 0.1 to 10 parts by weight, and further preferably 0.1 to 5 parts by weight per 100 parts by weight of the rubber component.

(Oil)

Examples of the oil include process oils and vegetable oils and fats. Examples of the process oil include paraffin-based process oils, naphthene-based process oils, aromatic-based process oils, MES (mild extracted solvate) oils, and TDAE (treated distilled aromatic extract) oils. Examples of commercially available products include aromatic oils ("NC-140" manufactured by Cosmo Oil Co., Ltd.), process oils ("Diana Process PS32" manufactured by Idemitsu Kosan Co., Ltd.), and TDAE oils ("VivaTec 500" manufactured by H&R Group).
When an oil is used, the amount of the oil is preferably 5 to 70 parts by weight and more preferably 20 to 60 parts by weight per 100 parts by weight of the rubber component.

(Wax)

Examples of the wax include "SUNNOC(R) wax" manufactured by Ouchi Shinko Chemical Industrial Co., Ltd. and "OZOACE-0355" manufactured by Nippon Seiro Co., Ltd.

(Resorcinol, resin)

In the present invention, resorcinol, resins such as resorcinol resins, modified resorcinol resins, cresol resins, modified cresol resins, phenol resins, and modified phenol resins may be used. Use of resorcinol or a resin thereof can improve the elongation at break and complex modulus of the vulcanized rubber composition.
Examples of resorcinol include resorcinol manufactured by SUMITOMO CHEMICAL COMPANY, LIMITED and the like. Examples of the resorcinol resin include resorcinol-formaldehyde condensates. Examples of the modified resorcinol resin include resorcinol resins in which repeating units are partially alkylated. Specific examples thereof include Penacolite resins B-18-S and B-20 manufactured by INDSPEC Chemical Corporation, SUMIKANOL 620 manufactured by Taoka Chemical Co., Ltd., R-6 manufactured by Uniroyal Chemical Co., SRF1501 manufactured by Schenectady Chemicals Inc., and Arofene 7209 manufactured by Ashland Inc.
Examples of the cresol resin include cresol-formaldehyde condensates. Examples of the modified cresol resin include cresol resins in which a methyl group at an end thereof is modified with a hydroxyl group and cresol resins in which repeating units are partially alkylated. Specific examples thereof include SUMIKANOL 610 manufactured by Taoka Chemical Co., Ltd. and PR-X11061 manufactured by Sumitomo Bakelite Co., Ltd.
Examples of the phenol resin include phenol-formaldehyde condensates. Examples of the modified phenol resin include resins obtained by modifying a phenol resin with cashew oil, tall oil, linseed oil, various vegetable oils, unsaturated fatty acids, rosin, alkylbenzene resins, aniline, melamine, or the like.
Examples of other resins include methoxylated methylolmelamine resins such as "SUMIKANOL 507AP" manufactured by SUMITOMO CHEMICAL COMPANY, LIMITED; coumarone-indene resins such as Coumarone resin NG4 manufactured by Nittetsu Chemical Industrial Co., Ltd. (softening point: 81 to 100°C) and "Process Resin AC5" manufactured by Kobe Oil Chemical Industrial Co., Ltd. (softening point: 75°C); terpene-based resins such as terpene resins, terpene-phenol resins, and aromatic modified terpene resins; rosin derivatives such as "NIKANOL(R) A70" manufactured by Mitsubishi Gas Chemical Company, Inc. (softening point: 70 to 90°C); hydrogenated rosin derivatives; novolac-type alkylphenol resins; resol-type alkylphenol resins ; C5-based petroleum resin; and liquid polybutadiene.

(Viscoelasticity improving agent)

Examples of the viscoelasticity improving agent include N,N'-bis(2-methyl-2-nitropropyl)-1,6-hexanediamine (e.g., "SUMIFINE(R) 1162" manufactured by SUMITOMO CHEMICAL COMPANY, LIMITED), dithiouracil compounds described in Japanese Patent Laid-Open No. 63-23942, "TACKIROL(R) AP" and "TACKIROL(R) V-200" manufactured by Taoka Chemical Co., Ltd., alkylphenol-sulfur chloride condensate described in Japanese Patent Laid-Open No. 2009-138148, 1,6-bis(dibenzylthiocarbamoyldithio)hexane (e.g., "KA9188" manufactured by Bayer AG), 1,6-hexamethylene dithiosulfate disodium salt dihydrate, 1,3-bis(citraconimide methyl)benzene (e.g., "Perkalink 900" manufactured by Flexsys), 1-benzoyl-2-phenyl hydrazide, carboxylic acid hydrazide derivatives such as 1-hydroxy-N'-(1-methylethylidene)-2-naphthoic acid hydrazide, 3-hydroxy-N'-(1-methylethylidene)-2-naphthoic acid hydrazide, 1-hydroxy-N'-(1-methylpropylidene)-2-naphthoic acid hydrazide described in Japanese Patent Laid-Open No. 2004-91505, 3-hydroxy-N'-(1-methylpropylidene)-2-naphthoic acid hydrazide, 1-hydroxy-N'-(1,3-dimethylbutylidene)-2-naphthoic acid hydrazide, 3-hydroxy-N'-(1,3-dimethylbutylidene)-2-naphthoic acid hydrazide, 1-hydroxy-N'-(2-furylmethylene)-2-naphthoic acid hydrazide, 3-hydroxy-N'-(2-furylmethylene)-2-naphthoic acid hydrazide, 3-hydroxy-N'-(1,3-dimethylbutylidene)-2-naphthoic acid hydrazide described in Japanese Patent Laid-Open No. 2000-190704, 3-hydroxy-N'-(1,3-diphenylethylidene)-2-naphthoic acid hydrazide, 3-hydroxy-N'-(1-methylethylidene)-2-naphthoic acid hydrazide, bismercaptooxadiazole compounds described in Japanese Patent Laid-Open No. 2006-328310, pyrithione salt compounds described in Japanese Patent Laid-Open No. 2009-40898, and cobalt hydroxide compounds described in Japanese Patent Laid-Open No. 2006-249361.

(Peptizing agent)

A peptizing agent is not particularly limited as long as it is usually used in the field of rubber. Examples thereof include aromatic mercaptan-based peptizing agents, aromatic disulfide-based peptizing agents, and aromatic mercaptan metal salt-based peptizing agents described on pages 446 to 449 in Gomu Kogyo Binran (Rubber Industry Handbook), 4th edition edited by Society of Rubber Industry, Japan. Among these, dixylyl disulfide and o,o'-dibenzamidodiphenyl disulfide ("NOCTIZER SS" manufactured by Ouchi Shinko Chemical Industrial Co., Ltd.) are preferred. One alone peptizing agent or two or more peptizing agents may be used either singly or as combined.

When a peptizing agent is used, the amount of the peptizing agent is preferably 0.01 to 1 part by weight and more preferably 0.05 to 0.5 parts by weight per 100 parts by weight of the rubber component.

(Retarder)

Examples of the retarder include phthalic anhydride, benzoic acid, salicylic acid, N-nitrosodiphenylamine, N-(cyclohexylthio)phthalimide (CTP), sulfonamide derivatives, diphenylurea, bis(tridecyl)pentaerythritol diphosphite, and include N-(cyclohexylthio)phthalimide (CTP).

When a retarder is used, the amount of the retarder is preferably 0.01 to 1 part by weight and more preferably 0.05 to 0.5 parts by weight per 100 parts by weight of the rubber component.

(Compound having oxyethylene units)

In the present invention, a compound having oxyethylene units having a structure represented by the formula: $-O-(CH_2-CH_2-O)_r-H$ [wherein r is an integer of 1 or more.] may be used. Here, in the above formula, r is preferably 2 or more and more preferably 3 or more. r is preferably 16 or less and more preferably 14 or less. When r is 17 or more, compatibility with a rubber component and reinforcing performance tend to decrease.

The position of the oxyethylene unit in a compound having oxyethylene units may be in the main chain, terminal, or side chain. From the viewpoint of the sustainability of the effect of preventing static electricity accumulation and reduction of electrical resistance on the surface of the resulting tire, among the compounds having oxyethylene units, a compound having oxyethylene units at least in the side chain is preferable.

Examples of a compound having oxyethylene units in the main chain include polyoxyethylene alkyl ethers, polyoxyethylene alkyl phenyl ethers, monoethylene glycol, diethylene glycol, triethylene glycol, polyoxyethylene sorbitan fatty acid esters, polyoxyethylene polyoxypropylene alkyl ethers, polyoxyethylene alkylamines, polyoxyethylene styrenated alkyl ethers, and polyoxyethylene alkylamides.

When a compound having oxyethylene units at least in the side chain is used, the number of oxyethylene units is preferably 4 or more, and more preferably 8 or more, per 100 carbon atoms constituting the main chain. When the number of oxyethylene units is 3 or less, the electrical resistance tends to increase. The number of oxyethylene units is preferably 12 or less and more preferably 10 or less. When the number of oxyethylene units is 13 or more, compatibility with a rubber component and reinforcing performance tend to decrease.

When a compound having oxyethylene units at least in the side chain is used, the main chain thereof is preferably mainly constituted of polyethylene, polypropylene or polystyrene.

<Production of vulcanized rubber composition>

The vulcanized rubber composition of the present invention may be produced by kneading a compound (I), a rubber component, and a sulfur component, and as required, a compound (II) and/or other components (e.g., silica, carbon black) and heating the resulting rubber composition. As described above, the compound (I) is considered to be consumed during the kneading or heating. Thus, when the compound (I) is used in the total amount of a predetermined amount and a predicted consumption amount (i.e., a predetermined amount + a predicted consumption amount), the vulcanized rubber composition of the present invention can be produced. The same applies to in the case where the compound (II) is used.

The rubber composition to be obtained by kneading the compound (I), a rubber component, and a sulfur

component (hereinafter, may be described as the "rubber composition containing a sulfur component") is preferably produced via the following steps 1 and 2, when a filler (e.g., silica, carbon black) is used:

step 1 of kneading a rubber component, a filler, and as required, other components, and
step 2 of kneading the rubber composition obtained in step 1, a sulfur component, and as required, other components.

A pre-kneading step for masticating the rubber component may be included before the step 1 to facilitate processing of the rubber component.
In the production of a rubber composition containing a sulfur component, the total amount of the compound (I) may be kneaded with a rubber component and the like in any of the pre-kneading step, step 1, and step 2, or the compound (I) may each be divided and kneaded with a rubber component and the like in at least two steps of the pre-kneading step to step 2. Alternatively, the compound (I) may be supported on the aforementioned filler in advance and then kneaded with a rubber component and the like.
When the compound (II) is used, in the production of a rubber composition containing a sulfur component, the total amount of the compound (II) may be kneaded with a rubber component and the like in any of the pre-kneading step, step 1, and step 2, or the compound (II) may each be divided and kneaded with a rubber component and the like in at least two steps of the pre-kneading step to step 2. Alternatively, the compound (II) may be supported on the aforementioned filler in advance and then kneaded with a rubber component and the like.
When zinc oxide is blended, zinc oxide is preferably kneaded with a rubber component and the like in step 1. When stearic acid is blended, stearic acid is preferably kneaded with a rubber component and the like in step 1. When a vulcanization accelerator is blended, the vulcanization accelerator is preferably kneaded with a rubber component and the like in step 2. When a peptizing agent is blended, the peptizing agent is preferably kneaded with a rubber component and the like in step 1. When a pre-kneading step is included, it is preferable to knead the total amount of the peptizing agent with a rubber component in the pre-kneading step or divide the peptizing agent and knead a part thereof with the rubber component in both the pre-kneading step and step 1. When a retarder is blended the retarder is preferably kneaded with a rubber component and the like in step 2.
For kneading in the step 1, for example, an internal mixer including a Banbury mixer, an open kneader, a pressure kneader, an extruder, an injection molding apparatus and the like can be used. The discharging temperature of the rubber composition after kneading in the step 1 is preferably 200°C or less and more preferably 120 to 180°C.
For kneading in the step 2, for example, an open roll, a calendar, and the like can be used. The kneading temperature (temperature of the rubber composition being kneaded) in the step 2 is preferably 60 to 120°C.
The vulcanized rubber composition of the present invention can be produced by vulcanizing a rubber composition containing a sulfur component. The vulcanized rubber composition of the present invention may be produced by processing a rubber composition containing a sulfur component into a particular shape and then vulcanizing the rubber composition.
The vulcanizing temperature is preferably 120 to 180°C. Those skilled in the art can appropriately determine the vulcanizing time according to the composition of the rubber composition. Vulcanization is generally performed under normal pressure or under pressure.

<Application>

The vulcanized rubber composition of the present invention is excellent in abrasion resistance and thus is preferably used in tires. Accordingly, the present invention provides a tire containing the vulcanized rubber composition.
Also, the vulcanized rubber composition of the present invention is preferably used in a tire member. Examples of the tire member include a tire belt member containing a vulcanized rubber composition of the present invention and a steel cord, a tire carcass member containing a vulcanized rubber composition of the present invention and a carcass fiber cord, a tire side wall member, a tire inner liner member, a tire cap tread member, and a tire under tread member.
The vulcanized rubber composition of the present invention can be used in various products (e.g., vibration-proof rubber, conveyor belt rubber, and engine mount rubber), in addition to tires and tire members.

Examples

While the present invention is more specifically described in the following by referring to Examples and the like, the present invention is not limited by the following Examples and the like. The present invention can be implemented by appropriately adding changes within the range compatible to the gist described above and below, and they are all included in the technical scope of the present invention.

Production Example 1: production of compound (IIa-1)

After addition of 500 mL of ethyl acetate to 21.5 g (0.15 mol) of 4,6-dimethyl-2-mercaptopyrimidine, 4,6-dimethyl-2-mercaptopyrimidine formed into blocks was crushed with ultrasonic waves (40°C, 30 minutes). After addition of 2.3 g (0.015 mol) of sodium iodide thereto, 14.4 mL (0.15 mol) of a 35% by weight hydrogen peroxide aqueous solution was added dropwise over 1 hour and 33 minutes, and then an exotherm was observed. After the total amount of the hydrogen peroxide aqueous solution was added dropwise, no complete solution was obtained, but a dispersion in which a solid was partially precipitated out was obtained. The dispersion was stirred at room temperature for 8 hours and 40 minutes, and disappearance of 4,6-dimethyl-2-mercaptopyrimidine was confirmed by TLC. Then, 100 mL of a saturated aqueous solution of sodium thiosulfate was added for quenching, and disappearance of hydrogen peroxide was confirmed with a peroxide testing strip. A precipitated solid was collected by suction filtration and then washed with water followed by ethyl acetate. A filtrate was transferred to a separatory funnel and extracted with 250 mL of ethyl acetate twice. Then, combined organic layers were washed with 100 mL of saturated brine and dried over sodium sulfate. After removal of the solid by filtration, a filtrate was concentrated with an evaporator to obtain a solid. The solid collected by suction filtration and the solid obtained by an evaporator were combined and dried under reduced pressure to obtain 21.0 g (yield: 98%) of a compound (IIa-1) (i.e., 2,2'-bis(4,6-dimethylpyrimidyl)disulfide) as a pale yellow solid. $^1$H-NMR ($CDCl_3$, 400 MHz) $\delta$ ppm: 2.39 (12H, s), 6.76 (2H, s)

Production Example 2: production of compound (IIa-2)

After addition of 500 mL of ethyl acetate to 22.9 g (0.20 mol) of 2-mercaptopyrimidine, 2-mercaptopyrimidine formed into blocks was crushed with ultrasonic waves (40°C, 30 minutes). After addition of 3.0 g (0.020 mol) of sodium iodide thereto, 19.4 mL (0.200 mol) of a 35% by weight hydrogen peroxide aqueous solution was added dropwise a room temperature over about 2 hours, and an exotherm occurred. After the total amount of the hydrogen peroxide aqueous solution was added dropwise, a complete solution was obtained. The solution was stirred at room temperature for 45 minutes, and disappearance of 2-mercaptopyrimidine was confirmed by TLC. Then, 100 mL of an aqueous solution containing 35.0 g (0.22 mol) of sodium thiosulfate was added to the solution for quenching, and disappearance of hydrogen peroxide was confirmed with a peroxide testing strip. A resulting solution was transferred to a separatory funnel and extracted with 150 mL of ethyl acetate twice. Then, combined organic layers were washed with 100 mL of saturated brine. The separated organic layer was dried over sodium sulfate, and the solid was removed by filtration. Then, a filtrate was concentrated by an evaporator and dried under reduced pressure to obtain 21.8 g (yield: 98%) of a compound (IIa-2) (i.e., 2,2'-dipyrimidyl disulfide) as a pale yellow.
$^1$H-NMR ($CDCl_3$, 400 MHz) $\delta$ ppm: 7.10 (4H, t, J = 4.8 Hz), 8.56 (2H, d, J = 4.8 Hz)

Example 1

<Step 1: Kneading by Banbury mixer>

Using a Banbury mixer (manufactured by Kobe Steel, Ltd., capacity: 1700 mL), 80 parts by weight of a styrene-butadiene copolymer rubber ("SBR TUFDENE 2000" manufactured by Asahi Kasei Corporation), 20 parts by weight of a butadiene rubber ("BR01" manufactured by JSR Corporation), 75 parts by weight of silica ("Nipsil(R) AQ" manufactured by Tosoh Silica Corporation, BET specific surface area :205 $m^2/g$), 5 parts by weight of carbon black HAF ("Asahi #70" manufactured by Asahi Carbon Co., Ltd.), 2 parts by weight of stearic acid, 3 parts by weight of zinc oxide, 1.5 parts by weight of an anti-aging agent (N-phenyl-N'-1,3-dimethylbutyl-p-phenylenediamine (6PPD), "Antigen(R) 6C" manufactured by SUMITOMO CHEMICAL COMPANY, LIMITED), 30 parts by weight of a TDAE oil ("VivaTec 500" manufactured by H&R Group), and 6 parts by weight of a compound that can be bonded to silica ("Si-75" manufactured by Evonik Industries AG), and 0.5 parts by weight of a compound (Ia-1) ("4,6-dimethyl-2-mercaptopyrimidine" manufactured by Tokyo Chemical Industry Co., Ltd.) were kneaded to obtain a rubber composition. In the step, all the components described above were placed in a Banbury mixer that was set at a temperature of 80°C and rotated at a rotor rotation speed of 25 rpm. Then, the mixture in the Banbury mixer was kneaded at a rotor rotation speed of 50 rpm for 3.5 minutes and further at a rotor rotation speed of 80 rpm for 1.5 minutes. The temperature of the rubber composition at the completion of kneading was 160 to 170°C.

<Step 2: Kneading by open roll machine>

The rubber composition obtained in the step 1, a vulcanization accelerator (1.5 parts by weight of N-cyclohexyl-2-benzothiazolylsulfenamide (CBS) and 2.0 parts by weight of diphenylguanidine (DPG)) and 2.0 parts by weight of a powder sulfur ("Fine powder sulfur" manufactured by Hosoi Chemical Industry Co., Ltd.) were kneaded in an open roll

machine at a roll setting temperature of 60°C to obtain a rubber composition.

<Vulcanization>

The rubber composition obtained in the step 2 was heated at 170°C for 12 minutes to thereby obtain a vulcanized rubber composition.

Examples 2 to 5 and Comparative Example 1

In the same manner as in Example 1 except that components in the kind and amount shown in Table 1 were used in the steps 1 and 2 and that the vulcanizing time for obtaining a vulcanized rubber composition was changed, vulcanized rubber compositions of Examples 2 to 5 and Comparative Example 1 were obtained.

The styrene-butadiene copolymer rubber and the like used were the same as those in Example 1. As the compound (IIa-1), that obtained in Production Example 1 was used.

The vulcanizing time was 12 minutes in Example 1 and Comparative Example 1, 25 minutes in Examples 2 and 5, and 60 minutes in Examples 3 and 4.

For evaluation of the abrasion resistance mentioned below, the amounts of the powder sulfur, CBS, and DPG were adjusted in Examples 1 to 5 such that the hardness of the vulcanized rubber compositions of Examples 1 to 5 was equivalent to the hardness of the vulcanized rubber composition of Comparative Example 1.

<Content of compound (I) and compound (II)>

(1) Extraction

A specimen (about 2 g) was taken from the vulcanized rubber composition obtained each of Examples 1 to 5. The weight of the specimen was weighed using a precision balance capable of measuring to 0.1 mg. The specimen was cut into pieces having a side length of 2 mm or less. The resulting pieces (about 2 g) were added in the extraction unit of a Soxhlet extractor, in which unit a cylindrical filter had been placed. Acetone (100 mL) was added to the receiving flask (capacity: 150 mL) of the Soxhlet extractor, and extraction was conducted under reflux conditions for 8 hours.

(2) Analysis

After the extraction, the extracted liquid in the receiving flask was concentrated as required. The total amount thereof was added to a volumetric flask of 20 to 100 mL. Acetone or any organic solvent was added to dilute the extracted liquid to obtain an analysis solution. The total amount of the analysis solution obtained by this dilution (i.e., the diluted extracted solution) is denoted by c. The resulting analysis solution (3 μL) was injected to a liquid chromatography (LC) apparatus ("LC 20A" manufactured by SHIMADZU CORPORATION) while an "L-column ODS (4.6 mmϕ × 150 mm, particle size: 5 μm)" was used as the column and 2 solutions (liquid A and liquid B) were used as the mobile phase. An aqueous solution of trifluoroacetic acid (concentration: 0.05% by weight) was used as the liquid A, and an acetonitrile solution of trifluoroacetic acid (concentration: 0.05% by weight) was used as the liquid B. The gradient conditions of the liquid B were set to "0 minute: 5% by volume, 0 to 40 minutes: the proportion of the liquid B in the mobile phase was changed at a constant rate from 5% by volume to 100% by volume, 40 to 45 minutes: 100% by volume, and 45.01 to 50 minutes: 5% by volume". LC analysis was conducted under conditions of column temperature: 40°C, mobile phase flow rate: 1.0 mL/minute, and detection wavelength: 254 nm, and the area value of the detected peak of the analysis solution was calculated.

The standard sample (3 μL) of each of the compound (I) and the compound (II) was subjected to LC analysis under the conditions described above in advance, and the position and area value of the detected peaks thereof were identified. The area value of the detected peak of the analysis solution, the amount of the vulcanized rubber composition (pieces) (about 2 g) used for the extraction, the concentration of the standard sample (a), the area value of the detected peak of the standard sample (b), and the total amount of the analysis solution (i.e., the diluted extracted solution) (c) of the compound (I) or the compound (II) were used to calculate the content of the compound (I) or the compound (II) with respect to the entire vulcanized rubber composition by the following expression. The results are shown in Table 1.

Content of compound (I) or compound (II) (% by weight) = (100 × area value of detected peak of analysis solution × a × c)/(amount of vulcanized rubber composition used for extraction (about 2 g) × b)

In the expression described above, a represents the concentration of the standard sample (g/mL), b represents the peak value of the detected peak of the standard sample, and c represents the total amount of the analysis solution (i.e., the diluted extracted solution) (mL).

<Evaluation of abrasion resistance>

A DIN abrasion tester AB-6111 (manufactured by Ueshima Seisakusho Co., Ltd.) was used to measure the abrasion volume (unit: $mm^3$) of the vulcanized rubber composition of each of Examples 1 to 13, each of which contains a predetermined amount of the compound (I), and the vulcanized rubber composition of Comparative Example 1, which does not contain the compound (I), in accordance with JIS K6264-2:2005 "Rubber, vulcanized or thermoplastic-Determination of abrasion resistance". The abrasion resistance of the vulcanized rubber composition of each of Examples 1 to 5 was calculated by the following expression:

Index of abrasion resistance = 100 × (abrasion volume of Comparative Example 1)/(abrasion volume of each of Examples 1 to 5). The results are shown in Table 1. The larger this index, the better the abrasion resistance.

[Table 1]

| | | Comparative Example 1 | Example 1 | Example 2 | Example 3 | Example 4 | Example 5 |
|---|---|---|---|---|---|---|---|
| Step 1 | Styrene-butadiene copolymer rubber (parts) | 80 | 80 | 80 | 80 | 80 | 80 |
| | Butadiene rubber (parts) | 20 | 20 | 20 | 20 | 20 | 20 |
| | Silica (parts) | 75 | 75 | 75 | 75 | 75 | 75 |
| | Carbon black (parts) | 5 | 5 | 5 | 5 | 5 | 5 |
| | Stearic acid (parts) | 2 | 2 | 2 | 2 | 2 | 2 |
| | Zinc oxide (parts) | 3 | 3 | 3 | 3 | 3 | 3 |
| | Anti-aging agent (parts) | 1.5 | 1.5 | 1.5 | 1.5 | 1.5 | 1.5 |
| | Compound (Ia-1) (parts) | - | 0.5 | 1 | 3 | 5 | 1 |
| | Compound (IIa-1) (parts) | - | - | - | - | - | 0.5 |
| | TDAE oil (parts) | 30 | 30 | 30 | 30 | 30 | 30 |
| | Compound that can be bonded to silica (parts) | 6 | 6 | 6 | 6 | 6 | 6 |
| Step 2 | Powder sulfur (parts) | 2 | 2 | 1.4 | 1 | 0.86 | 1.26 |
| | CBS (parts) | 1.5 | 1.5 | 1.05 | 0.75 | 0.645 | 0.945 |
| | DPG (parts) | 2 | 2 | 2 | 2 | 2 | 2 |
| Content of compound (I) (%) | | Not measured | 0.018 | 0.066 | 0.192 | 0.495 | 0.075 |
| Content of compound (II) (%) | | Not measured | - | - | - | - | 0.010 |
| Index of abrasion resistance | | - | 108 | 151 | 172 | 165 | 178 |
| (Note) parts = parts by weight, % = % by weight | | | | | | | |

As shown in Table 1, the vulcanized rubber compositions of Examples 1 to 5, which each contain a predetermined amount of compound (I), are excellent in abrasion resistance.

Example 6

<Step 1: Kneading by Labo Plastomill>

Using a Labo Plastomill (manufactured by Toyo Seiki Seisaku-sho, Ltd., capacity: 600 mL), 80 parts by weight of a styrene-butadiene copolymer rubber ("SBR TUFDENE 2000" manufactured by Asahi Kasei Corporation), 20 parts by weight of a butadiene rubber ("BR01" manufactured by JSR Corporation), 75 parts by weight of silica ("Nipsil(R) AQ" manufactured by Tosoh Silica Corporation, BET specific surface area: 205 $m^2/g$), 5 parts by weight of carbon black HAF ("Asahi #70" manufactured by Asahi Carbon Co., Ltd.), 2 parts by weight of stearic acid, 3 parts by weight of zinc oxide, 1.5 parts by weight of an anti-aging agent (N-phenyl-N’-1,3-dimethylbutyl-p-phenylenediamine (6PPD), "Antigen(R) 6C" manufactured by SUMITOMO CHEMICAL COMPANY, LIMITED), 30 parts by weight of a TDAE oil ("VivaTec 500" manufactured by H&R Group), and 6 parts by weight of a compound that can be bonded to silica ("Si-75" manufactured by Evonik Industries AG), and 2 parts by weight of a compound (Ia-2) ("2-mercaptopyrimidine" manufactured by Tokyo

Chemical Industry Co., Ltd.) were kneaded to obtain a rubber composition. In the step, all the components described above were placed in a Labo Plastomill that was set at a temperature of 140°C and rotated at a rotor rotation speed of 25 rpm. Then, the mixture in the Labo Plastomill was kneaded at a rotor rotation speed of 10 rpm for 3 minutes and further at a rotor rotation speed of 60 rpm for 5 minutes. The temperature of the rubber composition at the completion of kneading was 155 to 165°C.

<Step 2: Kneading by open roll machine>

The rubber composition obtained in the step 1, a vulcanization accelerator (1.5 parts by weight of N-cyclohexyl-2-benzothiazolylsulfenamide (CBS) and 2.0 parts by weight of diphenylguanidine (DPG)), and 2.0 parts by weight of a powder sulfur ("Fine powder sulfur" manufactured by Hosoi Chemical Industry Co., Ltd.) were kneaded in an open roll machine at a roll setting temperature of 60°C to obtain a rubber composition.

<Vulcanization>

The rubber composition obtained in the step 2 was heated at 170°C for 14 minutes to thereby obtain a vulcanized rubber composition.

Examples 7 to 12 and Comparative Example 2

In the same manner as in Example 6 except that components in the kind and amount shown in Tables 2 and 3 were used and that the vulcanizing time for obtaining a vulcanized rubber was changed, vulcanized rubber compositions of Examples 7 to 12 and Comparative Example 2 were obtained.

The styrene-butadiene copolymer rubber and the like used were the same as those in Example 6.

The vulcanizing time was 14 minutes in Examples 7, 10, and 11, 36 minutes in Examples 8 and 12, 21 minutes in Example 9, 20 minutes in Example 13, and 9 minutes in Comparative Example 2.

As the compound (Ia-2), "2-mercaptopyrimidine" manufactured by Tokyo Chemical Industry Co., Ltd. was used. As the compound (Ib-1), "2-mercaptopyridine" manufactured by Tokyo Chemical Industry Co., Ltd. was used. As the compound (Ib-2), "2-mercapto-5-nitropyridine" manufactured by Tokyo Chemical Industry Co., Ltd. was used. As the compound (Ic-1), "4-mercaptopyridine" manufactured by Tokyo Chemical Industry Co., Ltd. was used.

As the compound (IIa-2), that obtained in Production Example 2 was used. As the compound (IIb-1), "2,2'-dipyridyl disulfide" manufactured by Tokyo Chemical Industry Co., Ltd. was used. As the compound (IIb-2), "2,2'-dithiobis(5-nitropyridine)" manufactured by Tokyo Chemical Industry Co., Ltd. was used. As the compound (IIc-1), "4,4'-dipyridyl disulfide" manufactured by Tokyo Chemical Industry Co., Ltd. was used.

<Content of compound (I) and compound (II)>

In the same manner as in Example 1 and the like, the content of each of the compound (I) and the compound (II) in the vulcanized rubber compositions obtained in Examples 6 to 12 was measured and calculated. The results are shown in Tables 2 and 3.

<Evaluation of abrasion resistance>

The hardness of the vulcanized rubber composition of Example 8 was equivalent to the hardness of the vulcanized rubber composition of Comparative Example 1. Thus, in the same manner as in Example 1 and the like, the index of the abrasion resistance of the vulcanized rubber composition was calculated. The index of abrasion resistance of the vulcanized rubber composition of Example 8 was calculated by the following expression:

Index of abrasion resistance = 100 × (abrasion volume of Comparative Example 1)/(abrasion volume of Example 8). The results are shown in the following Table 2.

The hardness of the vulcanized rubber composition obtained in each of Examples 6, 7 and 9 to 12 was not equivalent to the hardness of the vulcanized rubber composition of Comparative Example 1. Thus, Comparative Example 2 was conducted with the amount of each of the powder sulfur, CBS, and DPG adjusted so as to obtain a vulcanized

rubber composition having a hardness equivalent to that of Example 6 and the like. Then, in the same manner as in Example 1 and the like, the abrasion volume of the vulcanized rubber composition (unit: $mm^3$) was measured, and the index of the abrasion resistance of the vulcanized rubber composition of each of Examples 6, 7 and 9 to 12 was calculated by the following formula:

Index of abrasion resistance = 100 × (abrasion volume of Comparative Example 2)/(abrasion volume of each of Examples 6, 7 and 9 to 12). The results are shown in the following Table 3.

[Table 2]

| | | Comparative Example 1 | Example 8 |
|---|---|---|---|
| Step 1 | Styrene-butadiene copolymer rubber (parts) | 80 | 80 |
| | Butadiene rubber (parts) | 20 | 20 |
| | Silica (parts) | 75 | 75 |
| | Carbon black (parts) | 5 | 5 |
| | Stearic acid (parts) | 2 | 2 |
| | Zinc oxide (parts) | 3 | 3 |
| | Anti-aging agent (parts) | 1.5 | 1.5 |
| | Compound (Ic-1) (parts) - 2 | | |
| | TDAE oil (parts) | 30 | 30 |
| | Compound that can be bonded to silica (parts) | 6 | 6 |
| Step 2 | Powder sulfur (parts) | 2 | 2 |
| | CBS (parts) | 1.5 | 1.5 |
| | DPG (parts) | 2 | 2 |
| Content of compound (I) (%) | | Not measured | 0.010 |
| Content of compound (II) (%) | | Not measured | - |
| Index of abrasion resistance | | - | 203 |
| (Note) parts = parts by weight, % = % by weight | | | |

[Table 3]

| | | Comparative Example 2 | Example 6 | Example 7 | Example 9 | Example 10 | Example 11 | Example 12 |
|---|---|---|---|---|---|---|---|---|
| Step 1 | Styrene-butadiene copolymer rubber (parts) | 80 | 80 | 80 | 80 | 80 | 80 | 80 |
| | Butadiene rubber (parts) | 20 | 20 | 20 | 20 | 20 | 20 | 20 |
| | Silica (parts) | 75 | 75 | 75 | 75 | 75 | 75 | 75 |
| | Carbon black (parts) | 5 | 5 | 5 | 5 | 5 | 5 | 5 |
| | Stearic acid (parts) | 2 | 2 | 2 | 2 | 2 | 2 | 2 |
| | Zinc oxide (parts) | 3 | 3 | 3 | 3 | 3 | 3 | 3 |
| | Anti-aging agent (parts) | 1.5 | 1.5 | 1.5 | 1.5 | 1.5 | 1.5 | 1.5 |
| | Compound (Ia-2) (parts) | - | 2 | - | 2 | - | - | - |
| | Compound (IIa-2) (parts) | - | - | - | 1 | - | - | - |
| | Compound (Ib-1) (parts) | - | - | - | - | 2 | - | - |
| | Compound (IIb-1) (parts) | - | - | - | - | 1 | - | - |
| | Compound (Ib-2) (parts) | - | - | 2 | - | - | 2 | - |
| | Compound (IIb-2) (parts) | - | - | - | - | - | 1 | - |
| | Compound (Ic-1) (parts) | - | - | - | - | - | - | 2 |
| | Compound (IIc-1) (parts) | - | - | - | - | - | - | 1 |
| | TDAE oil (parts) | 30 | 30 | 30 | 30 | 30 | 30 | 30 |
| | Compound that can be bonded to silica (parts) | 6 | 6 | 6 | 6 | 6 | 6 | 6 |
| Step 2 | Powder sulfur (parts) | 3 | 2 | 0.86 | 2 | 2 | 2 | 0.86 |
| | CBS (parts) | 2 | 1.5 | 0.65 | 1.5 | 1.5 | 1.5 | 0.65 |
| | DPG (parts) | 2 | 2 | 2 | 2 | 2 | 2 | 2 |
| Content of compound (I) (%) | | Not measured | 0.0001 | 0.188 | 0.0008 | 0.074 | 0.273 | 0.007 |

(continued)

| | Comparative Example 2 | Example 6 | Example 7 | Example 9 | Example 10 | Example 11 | Example 12 |
|---|---|---|---|---|---|---|---|
| Content of compound (II) (%) | Not measured | - | - | 0.031 | 0.042 | 0.0013 | 0.070 |
| Index of abrasion resistance - 182 237 178 184 359 159 | | | | | | | |
| (Note) parts = parts by weight, % = % by weight | | | | | | | |

As shown in Tables 2 and 3, the vulcanized rubber compositions of Examples 6 to 12, which each contain a predetermined amount of compound (I), are excellent in abrasion resistance.

Example 13 and Comparative Example 3

In the same manner as in Example 1 except that components in the kind and amount shown in Table 4 were used in the steps 1 and 2 of Example 1 and that the vulcanizing time for obtaining a vulcanized rubber was changed, vulcanized rubber compositions of Example 13 and Comparative Example 3 were obtained.

The styrene-butadiene copolymer rubber and the like used were the same as those in Example 1. As the compound (IIa-1), that obtained in Production Example 1 was used.

The vulcanizing time was 48 minutes in Example 13 and 9 minutes in Comparative Example 9.

<Content of compound (I) and compound (II)>

In the same manner as in Example 1 and the like, the content of each of the compound (I) and the compound (II) in the vulcanized rubber composition obtained in Example 13 was measured and calculated. The results are shown in Table 4.

<Evaluation of abrasion resistance>

The hardness of the vulcanized rubber composition obtained in Example 13 was not equivalent to the hardness of the vulcanized rubber composition of each of Comparative Examples 1 and 2. Thus, Comparative Example 3 was conducted with the amount of each of the powder sulfur, CBS, and DPG adjusted so as to obtain a vulcanized rubber composition having a hardness equivalent to that of Example 13. Then, in the same manner as in Example 1 and the like, the abrasion volume of the vulcanized rubber composition (unit: $mm^3$) was measured, and the index of the abrasion resistance of the vulcanized rubber composition of Example 13 was calculated by the following formula:

Index of abrasion resistance = 100 × (abrasion volume of Comparative Example 3)/(abrasion volume of Example 13). The results are shown in Table 4.

[Table 4]

| | | Comparative Example 3 | Example 13 |
|---|---|---|---|
| Step 1 | Styrene-butadiene copolymer rubber (parts) | 80 | 80 |
| | Butadiene rubber (parts) | 20 | 20 |
| | Silica (parts) | 75 | 75 |
| | Carbon black (parts) | 5 | 5 |
| | Stearic acid (parts) | 2 | 2 |
| | Zinc oxide (parts) | 3 | 3 |
| | Anti-aging agent (parts) | 1.5 | 1.5 |
| | Compound (Ia-1) (parts) | - | 2 |
| | Compound (IIa-1) (parts) | - | 5 |
| | TDAE oil (parts) | 30 | 30 |
| | Compound that can be bonded to silica (parts) | 6 | 6 |
| Step 2 | Powder sulfur (parts) | 4 | 2 |
| | CBS (parts) | 3 | 2 |
| | DPG (parts) | 2 | 2 |
| Content of compound (I) (%) | | Not measured | 0.436 |
| Content of compound (II) (%) | | Not measured | 0.031 |
| Index of abrasion resistance | | - | 512 |
| (Note) parts = parts by weight, % = % by weight | | | |

As shown in Table 4, the vulcanized rubber composition of Example 13, which contains a predetermined amount of compound (I), is excellent in abrasion resistance.

Example 14 and Comparative Example 4

In the same manner as in Example 1 except that components in the kind and amount shown in Table 5 were used in the steps 1 and 2 of Example 1 and that the vulcanizing time for obtaining a vulcanized rubber was changed, vulcanized rubber compositions of Example 14 and Comparative Example 4 were obtained.

As the styrene-butadiene copolymer rubber, "Nipol NS616" manufactured by Nippon Zeon Co., Ltd. was used. The butadiene rubber and the like used were the same as those in Example 1. Further, as the compound (IIa-1), that obtained in Production Example 1 was used.

The vulcanizing time was 60 minutes in Example 14 and 40 minutes in Comparative Example 4.

<Content of compound (I) and compound (II)>

In the same manner as in Example 1 and the like, the content of each of the compound (I) and the compound (II) in the vulcanized rubber composition obtained in Example 14 was measured and calculated. The results are shown in Table 5.

<Evaluation of abrasion resistance>

The hardness of the vulcanized rubber composition obtained in Example 14 was not equivalent to the hardness of the vulcanized rubber composition of Comparative Example 1. Thus, Comparative Example 4 was conducted with the amount of each of the powder sulfur, CBS, and DPG adjusted so as to obtain a vulcanized rubber composition having a hardness equivalent to that of Example 14. Then, in the same manner as in Example 1 and the like, the abrasion volume of the vulcanized rubber composition (unit: $mm^3$) was measured, and the index of the abrasion resistance of the vulcanized rubber composition of Example 4 was calculated by the following formula:

Index of abrasion resistance = 100 × (abrasion volume of Comparative Example 4)/(abrasion volume of Example 14).

The results are shown in the following Table 5.

[Table 5]

| | | Comparative Example 4 | Example 14 |
|---|---|---|---|
| Step 1 | Styrene-butadiene copolymer rubber (parts) | 80 | 80 |
| | Butadiene rubber (parts) | 20 | 20 |
| | Silica (parts) | 75 | 75 |
| | Carbon black (parts) | 5 | 5 |
| | Stearic acid (parts) | 2 | 2 |
| | Zinc oxide (parts) | 3 | 3 |
| | Anti-aging agent (parts) | 1.5 | 1.5 |
| | Compound (Ia-1) (parts) | - | 1 |
| | Compound (IIa-1) (parts) | - | 0.5 |
| | TDAE oil (parts) | 30 | 30 |
| | Compound that can be bonded to silica (parts) | 6 | 6 |
| Step 2 | Powder sulfur (parts) | 2.0 | 1.3 |
| | CBS (parts) | 1.5 | 1 |
| | DPG (parts) | 2.0 | 2 |
| Content of compound (I) (%) | | Not measured | 0.021 |
| Content of compound (II) (%) | | Not measured | 0.009 |
| Index of abrasion resistance | | - | 148 |
| (Note) parts = parts by weight, % = % by weight | | | |

As shown in Table 5, the vulcanized rubber composition of Example 14, which contains a predetermined amount of compound (I), is excellent in abrasion resistance.

Example 15 and Comparative Example 5

In the same manner as in Example 1 except that components in the kind and amount shown in Table 6 were used in the steps 1 and 2 of Example 1 and that the vulcanizing time for obtaining a vulcanized rubber was changed, vulcanized rubber compositions of Example 15 and Comparative Example 5 were obtained.

As the styrene-butadiene copolymer rubber, "SBR TUFDENE 3835" manufactured by Asahi Kasei Corporation was used. The butadiene rubber and the like used were the same as those in Example 1.

The vulcanizing time was 15 minutes in Example 15 and Comparative Example 5.

<Content of compound (I)>

In the same manner as in Example 1 and the like, the content of the compound (I) in the vulcanized rubber composition obtained in Example 15 was measured and calculated. The results are shown in Table 6.

<Evaluation of abrasion resistance>

The hardness of the vulcanized rubber composition obtained in Example 15 was not equivalent to the hardness of the vulcanized rubber composition of Comparative Example 1. Thus, Comparative Example 5 was conducted with the amount of each of the powder sulfur, CBS, and DPG adjusted so as to obtain a vulcanized rubber composition having a hardness equivalent to that of Example 15. Then, in the same manner as in Example 1 and the like, the abrasion volume of the vulcanized rubber composition (unit: $mm^3$) was measured, and the index of the abrasion resistance of the vulcanized rubber composition of Example 15 was calculated by the following formula:

Index of abrasion resistance = 100 × (abrasion volume of Comparative Example 5)/(abrasion volume of Example 15). The results are shown in Table 6.

[Table 6]

| | | Comparative Example 5 | Example 15 |
|---|---|---|---|
| Step 1 | Styrene-butadiene copolymer rubber (parts) | 80 | 80 |
| | Butadiene rubber (parts) | 20 | 20 |
| | Silica (parts) | 75 | 75 |
| | Carbon black (parts) | 5 | 5 |
| | Stearic acid (parts) | 2 | 2 |
| | Zinc oxide (parts) | 3 | 3 |
| | Anti-aging agent (parts) | 1.5 | 1.5 |
| | Compound (Ia-1) (parts) | - 2 | |
| | TDAE oil (parts) | 30 | 30 |
| | Compound that can be bonded to silica (parts) | 6 | 6 |
| Step 2 | Powder sulfur (parts) | 2.0 | 1.1 |
| | CBS (parts) | 1.5 | 0.8 |
| | DPG (parts) | 2.0 | 2.0 |
| Content of compound (I) (%) | | Not measured | 0.021 |
| Index of abrasion resistance | | - | 112 |
| (Note) parts = parts by weight, % = % by weight | | | |

As shown in Table 6, the vulcanized rubber composition of Example 15, which contains a predetermined amount of compound (I), is excellent in abrasion resistance.

Example 6 and Comparative Example 6

In the same manner as in Example 1 except that components in the kind and amount shown in Table 7 were used in the steps 1 and 2 of Example 1 and that the vulcanizing time for obtaining a vulcanized rubber was changed, vulcanized rubber compositions of Example 16 and Comparative Example 6 were obtained.

As the styrene-butadiene copolymer rubber, "Nipol NS612" manufactured by Nippon Zeon Co., Ltd. was used. The butadiene rubber and the like used were the same as those in Example 1. As the compound (Ib-1), "2-mercaptopyridine" manufactured by Tokyo Chemical Industry Co., Ltd. was used.

The vulcanizing time was 15 minutes in Example 16 and Comparative Example 6.

<Content of compound (I)>

In the same manner as in Example 1 and the like, the content of the compound (I) in the vulcanized rubber composition obtained in Example 16 was measured and calculated. The results are shown in Table 7.

<Evaluation of abrasion resistance>

The hardness of the vulcanized rubber composition obtained in Example 16 was not equivalent to the hardness of the vulcanized rubber composition of Comparative Example 1. Thus, Comparative Example 6 was conducted with the amount of each of the powder sulfur, CBS, and DPG adjusted so as to obtain a vulcanized rubber composition having a hardness equivalent to that of Example 16. Then, in the same manner as in Example 1 and the like, the abrasion volume of the vulcanized rubber composition (unit: $mm^3$) was measured, and the index of the abrasion resistance of the vulcanized rubber composition of Example 16 was calculated by the following formula:

Index of abrasion resistance = 100 × (abrasion volume of Comparative Example 6)/(abrasion volume of Example 16). The results are shown in Table 7.

[Table 7]

| | | Comparative Example 6 | Example 16 |
|---|---|---|---|
| | Styrene-butadiene copolymer rubber (parts) | 60 | 60 |
| | Butadiene rubber (parts) | 40 | 40 |
| | Silica (parts) | 75 | 75 |
| | Carbon black (parts) | 5 | 5 |
| | Stearic acid (parts) | 2 | 2 |
| | Zinc oxide (parts) | 3 | 3 |
| | Anti-aging agent (parts) | 1.5 | 1.5 |
| | Compound (Ib-1) (parts) | - 2 | |
| | TDAE oil (parts) | 30 | 30 |
| | Compound that can be bonded to silica (parts) | 6 | 6 |
| Step 2 | Powder sulfur (parts) | 2.0 | 1.1 |
| | CBS (parts) | 1.5 | 0.8 |
| | DPG (parts) | 2.0 | 2.0 |
| Content of compound (I) (%) | | Not measured | 0.011 |
| Index of abrasion resistance | | - | 172 |
| (Note) parts = parts by weight, % = % by weight | | | |

As shown in Table 7, the vulcanized rubber composition of Example 16, which contains a predetermined amount of compound (I), is excellent in abrasion resistance.

Example 17 and Comparative Example 7

In the same manner as in Example 1 except that components in the kind and amount shown in Table 8 were used in the steps 1 and 2 of Example 1 and that the vulcanizing time for obtaining a vulcanized rubber was changed, vulcanized rubber compositions of Example 17 and Comparative Example 7 were obtained.

As the styrene-butadiene copolymer rubber, "Nipol NS540" manufactured by Nippon Zeon Co., Ltd. was used.

The butadiene rubber and the like used were the same as those in Example 1. As the compound (Ib-1), "2-mercaptopyridine" manufactured by Tokyo Chemical Industry Co., Ltd. was used. As the compound (IIb-1), "2,2'-dipyridyl disulfide" manufactured by Tokyo Chemical Industry Co., Ltd. was used.

The vulcanizing time was 15 minutes in Example 17 and Comparative Example 7.

<Content of compound (I) and compound (II)>

In the same manner as in Example 1 and the like, the content of each of the compound (I) and the compound (II) in the vulcanized rubber composition obtained in Example 17 was measured and calculated. The results are shown in Table 8.

<Evaluation of abrasion resistance>

The hardness of the vulcanized rubber composition obtained in Example 17 was not equivalent to the hardness of the vulcanized rubber composition of Comparative Example 1. Thus, Comparative Example 7 was conducted with the amount of each of the powder sulfur, CBS, and DPG adjusted so as to obtain a vulcanized rubber composition having a hardness equivalent to that of Example 17. Then, in the same manner as in Example 1 and the like, the abrasion volume of the vulcanized rubber composition (unit: $mm^3$) was measured, and the index of the abrasion resistance of the vulcanized rubber composition of Example 17 was calculated by the following formula:

$$\text{Index of abrasion resistance} = 100 \times (\text{abrasion volume of Comparative Example 7})/(\text{abrasion volume of Example 17}).$$

The results are shown in Table 8.

[Table 8]

| | | Comparative Example 7 | Example 17 |
|---|---|---|---|
| Step 1 | Styrene-butadiene copolymer rubber (parts) | 80 | 80 |
| | Butadiene rubber (parts) | 20 | 20 |
| | Silica (parts) | 75 | 75 |
| | Carbon black (parts) | 5 | 5 |
| | Stearic acid (parts) | 2 | 2 |
| | Zinc oxide (parts) | 3 | 3 |
| | Anti-aging agent (parts) | 1.5 | 1.5 |
| | Compound (Ib-1) (parts) | - | 2 |
| | Compound (IIb-1) (parts) | - | 1 |
| | TDAE oil (parts) | 30 | 30 |
| | Compound that can be bonded to silica (parts) | 6 | 6 |
| Step 2 | Powder sulfur (parts) | 2.0 | 2.6 |
| | CBS (parts) | 1.5 | 2 |
| | DPG (parts) | 2.0 | 2 |
| Content of compound (I) (%) | | Not measured | 0.007 |
| Content of compound (II) (%) | | Not measured | 0.328 |
| Index of abrasion resistance | | - | 129 |
| (Note) parts = parts by weight, % = % by weight | | | |

As shown in Table 8, the vulcanized rubber composition of Example 17, which contains a predetermined amount of compound (I), is excellent in abrasion resistance.

Examples 18 to 21

The components other than the sulfur, vulcanization accelerating aid, and vulcanization accelerator shown in Table 9 in the amounts shown in Table 9 are kneaded using a Banbury mixer at 165°C for 4 minutes to obtain a rubber composition.

To the rubber composition obtained as described above, the sulfur, vulcanization accelerating aid, and vulcanization accelerator shown in Table 9 in the amounts shown in Table 9 are added and kneaded using an open roll at 80°C for 4 minutes to obtain a rubber composition. The rubber composition is vulcanized for an appropriate vulcanizing time to obtain a vulcanized rubber composition.

[Table 9]

| | | Example 18 | Example 19 | Example 20 | Example 21 |
|---|---|---|---|---|---|
| Step 1 | Styrene-butadiene copolymer rubber 1 (parts) | 100 | - | - | - |
| | Styrene-butadiene copolymer rubber 2 (parts) | - | 80 | - | - |
| | Styrene-butadiene copolymer rubber 3 (parts) | - | - | 60 | - |
| | Butadiene rubber (parts) | - | 20 | 30 | - |
| | Natural rubber (parts) | - | - | 10 | 100 |
| | Silica (parts) | 10 | - | 75 | - |
| | Carbon black (parts) | 70 | 45 | 5 | 45 |
| | Stearic acid (parts) | 2 | 2 | 2 | 3 |
| | Zinc oxide (parts) | 3 | 3 | 3 | 5 |
| | Anti-aging agent (parts) | 1.5 | 1.5 | 1.5 | 1 |
| | Compound (Ia-1) (parts) | 2 | 2 | 1 | 2 |
| | Compound (IIa-1) (parts) | 1 | - | 0.5 | 1 |
| | TDAE oil (parts) | 30 | 30 | 30 | - |
| | Compound that can be bonded to silica (parts) | 0.8 | - | 6 | - |
| Step 2 | Powder sulfur (parts) | 2.6 | 1.1 | 1.30 | 2.6 |
| | CBS (parts) | 2 | 0.8 | 0.95 | 2 |
| | DPG (parts) | 2 | 2 | 2 | - |
| (Note) parts = parts by weight | | | | | |

The components shown in Table 9 are as follows.

Styrene-butadiene copolymer rubber 1: "SBR1502" manufactured by JSR Corporation

Styrene-butadiene copolymer rubber 2: "SBR TUFDENE 3835" manufactured by Asahi Kasei Corporation

Styrene-butadiene copolymer rubber 3: "Nipsol NS540" manufactured by Nippon Zeon Co., Ltd.

Butadiene rubber: "BR130B" manufactured by Ube Industries, Ltd.

Natural rubber: RSS#3

Industrial Applicability

The vulcanized rubber composition of the present invention is excellent in abrasion resistance and useful for

producing tires and the like.

The present application is based on Japanese Patent Application No. 2019-034798 filed in Japan, the entire contents of which are incorporated herein.

## Claims

1. A vulcanized rubber composition comprising a compound represented by the formula (I):

[Formula 1]

(I)

[wherein,

$X^{1a}$ represents a nitrogen atom or C-$R^{1a}$,
$X^{3a}$ represents a nitrogen atom or C-$R^{3a}$,
$X^{5a}$ represents a nitrogen atom or C-$R^{5a}$,
at least one of $X^{1a}$, $X^{3a}$, and $X^{5a}$ is a nitrogen atom,
and
$R^{1a}$ and $R^{3a}$ to $R^{6a}$ each independently represent a hydrogen atom, a halogen atom, a $C_{1-18}$ alkyl group optionally having a substituent, a $C_{3-10}$ cycloalkyl group optionally having a substituent, a $C_{6-18}$ aryl group optionally having a substituent, a $C_{7-20}$ aralkyl group optionally having a substituent, a carboxy group, a $C_{1-18}$ alkoxy-carbonyl group optionally having a substituent, a $C_{3-10}$ cycloalkyloxy-carbonyl group optionally having a substituent, a $C_{6-18}$ aryloxy-carbonyl group optionally having a substituent, a $C_{7-20}$ aralkyloxy-carbonyl group optionally having a substituent, a carbamoyl group optionally having a substituent, a hydroxy group, a $C_{1-18}$ alkoxy group optionally having a substituent, a $C_{3-10}$ cycloalkyloxy group optionally having a substituent, a $C_{6-18}$ aryloxy group optionally having a substituent, a $C_{7-20}$ aralkyloxy group optionally having a substituent, a $C_{1-18}$ alkyl-carbonyloxy group optionally having a substituent, a $C_{3-10}$ cycloalkyl-carbonyloxy group optionally having a substituent, a $C_{6-18}$ aryl-carbonyloxy group optionally having a substituent, a $C_{7-20}$ aralkyl-carbonyloxy group optionally having a substituent, an amino group optionally having a substituent, or a nitro group.] and a vulcanized rubber, wherein a content of the compound represented by the formula (I) is 0.00005 to 5% by weight with respect to the entire vulcanized rubber composition.

2. The vulcanized rubber composition according to claim 1, wherein $R^{1a}$ and $R^{3a}$ to $R^{6a}$ are each independently a hydrogen atom, a $C_{1-18}$ alkyl group optionally having a substituent, a carboxy group, a $C_{1-18}$ alkoxy-carbonyl group optionally having a substituent, or a nitro group.

3. The vulcanized rubber composition according to claim 1, wherein

both $X^{1a}$ and $X^{3a}$ are nitrogen atoms,
$X^{5a}$ is C-$R^{5a}$,
$R^{4a}$ and $R^{6a}$ are each independently a $C_{1-18}$ alkyl group optionally having a substituent, and
$R^{5a}$ is a hydrogen atom.

4. The vulcanized rubber composition according to any one of claims 1 to 3, further comprising a compound represented by the formula (II):

[Formula 2]

$$\text{(II)}$$

[wherein,

$X^{1b}$ represents a nitrogen atom or C-$R^{1b}$,
$X^{3b}$ represents a nitrogen atom or C-$R^{3b}$,
$X^{5b}$ represents a nitrogen atom or C-$R^{5b}$,
$X^{1c}$ represents a nitrogen atom or C-$R^{1c}$,
$X^{3c}$ represents a nitrogen atom or C-$R^{3c}$,
$X^{5c}$ represents a nitrogen atom or C-$R^{5c}$,
at least one of $X^{1b}$, $X^{3b}$, $X^{5b}$, $X^{1c}$, $X^{3c}$, and $X^{5c}$ is a nitrogen atom, and
$R^{1b}$ and $R^{3b}$ to R6 and $R^{1c}$ and $R^{3c}$ to $R^{6c}$ each independently represent a hydrogen atom, a halogen atom, a $C_{1-18}$ alkyl group optionally having a substituent, a $C_{3-10}$ cycloalkyl group optionally having a substituent, a $C_{6-18}$ aryl group optionally having a substituent, a $C_{7-20}$ aralkyl group optionally having a substituent, a carboxy group, a $C_{1-18}$ alkoxy-carbonyl group optionally having a substituent, a $C_{3-10}$ cycloalkyloxy-carbonyl group optionally having a substituent, a $C_{6-18}$ aryloxy-carbonyl group optionally having a substituent, a $C_{7-20}$ aralkyloxy-carbonyl group optionally having a substituent, a carbamoyl group optionally having a substituent, a hydroxy group, a $C_{1-18}$ alkoxy group optionally having a substituent, a $C_{3-10}$ cycloalkyloxy group optionally having a substituent, a $C_{6-18}$ aryloxy group optionally having a substituent, a $C_{7-20}$ aralkyloxy group optionally having a substituent, a $C_{1-18}$ alkyl-carbonyloxy group optionally having a substituent, a $C_{3-10}$ cycloalkyl-carbonyloxy group optionally having a substituent, a $C_{6-18}$ aryl-carbonyloxy group optionally having a substituent, a $C_{7-20}$ aralkyl-carbonyloxy group optionally having a substituent, an amino group optionally having a substituent, or a nitro group] at a content of 0.0001 to 1.0% by weight with respect to the entire vulcanized rubber composition.

**5.** The vulcanized rubber composition according to claim 4, wherein $R^{1b}$ and $R^{3b}$ to $R^{6b}$ and $R^{1c}$ and $R^{3c}$ to $R^{6c}$ are each independently a hydrogen atom, a $C_{1-18}$ alkyl group optionally having a substituent, a carboxy group, a $C_{1-18}$ alkoxy-carbonyl group optionally having a substituent, or a nitro group.

**6.** The vulcanized rubber composition according to claim 4, wherein
$X^{1b}$, $X^{3b}$, $X^{1c}$, and $X^{3c}$ are all nitrogen atoms,
$X^{5b}$ is C-$R^{5b}$,
$X^{5c}$ is C-$R^{5c}$,
$R^{4b}$, $R^{6b}$, $R^{4c}$, and $R^{6c}$ are each independently a $C_{1-18}$ alkyl group optionally having a substituent, and
both $R^{5b}$ and $R^{5c}$ are hydrogen atoms.

**7.** A tire comprising the vulcanized rubber composition according to any one of claims 1 to 6.

## INTERNATIONAL SEARCH REPORT

International application No. PCT/JP2020/006962

A. CLASSIFICATION OF SUBJECT MATTER

B60C 1/00(2006.01)i; C08K 5/372(2006.01)i; C08K 5/378(2006.01)i; C08L 21/00(2006.01)i

FI: C08L21/00; C08K5/378; C08K5/372; B60C1/00 Z

According to International Patent Classification (IPC) or to both national classification and IPC

B. FIELDS SEARCHED

Minimum documentation searched (classification system followed by classification symbols)

B60C1/00; C08K5/372; C08K5/378; C08L21/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 4-68042 A (BRIDGESTONE CORPORATION) 03.03.1992 (1992-03-03) claim 1, examples 1-3 | 1,2,4,5,7<br>3,6 |
| X<br>A | JP 2016-30762 A (BRIDGESTONE CORPORATION) 07.03.2016 (2016-03-07) claim 1, comparative examples 2, 5, paragraph [0078] | 1,2,7<br>3-6 |
| X<br>A | WO 2013/164912 A1 (BRIDGESTONE CORPORATION) 07.11.2013 (2013-11-07) claims 1, 9, 10, paragraph [0051], examples 9, 21, 33, 45, 57, 69, 81, 93 | 1-3,7<br>4-6 |
| X<br>A | WO 2006/057343 A1 (BRIDGESTONE CORPORATION) 01.06.2006 (2006-06-01) claims 1, 7, 8, paragraph [0022], examples 3, 4, 9, 10 | 1-3,7<br>4-6 |

☒ Further documents are listed in the continuation of Box C. ☒ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 01 May 2020 (01.05.2020) | 19 May 2020 (19.05.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2020/006962

C (Continuation). DOCUMENTS CONSIDERED TO BE RELEVANT

| Category* | Citation of document, with indication, where appropriate, of the relevant passages | Relevant to claim No. |
|---|---|---|
| X<br>A | US 3510443 A (HERCULES INCORPORATED) 05.05.1970 (1970-05-05) claim 1, examples | 1,2,7<br>3-6 |
| X<br>A | US 3341491 A (HERCULES INCORPORATED) 12.09.1967 (1967-09-12) claims 12, 18, examples | 1,2,7<br>3-6 |
| A | JP 2010-18716 A (TOYO TIRE AND RUBBER CO., LTD.) 28.01.2010 (2010-01-28) claims 1-3, example 5 | 1-7 |
| A | WO 01/016227 A1 (FLEXSYS B. V.) 08.03.2001 (2001-03-08) tables 1, 3, 4 | 1-7 |

Form PCT/ISA/210 (continuation of second sheet) (January 2015)

**INTERNATIONAL SEARCH REPORT**
Information on patent family members

International application No.
PCT/JP2020/006962

| Patent Documents referred in the Report | Publication Date | Patent Family | Publication Date |
|---|---|---|---|
| JP 4-68042 A | 03 Mar. 1992 | (Family: none) | |
| JP 2016-30762 A | 07 Mar.2016 | (Family: none) | |
| WO 2013/164912 A1 | 07 Nov. 2013 | US 2013/0296493 A1 claims 1, 9, 10, examples 9, 21, 33, 45, 57, 69, 81, 93<br>EP 2845869 A1<br>CN 104271624 A | |
| WO 2006/057343 A1 | 01 Jun. 2006 | US 2009/0227742 A1 claims 1, 7, 8 examples 3, 4, 9, 10<br>EP 1816144 A1<br>CN 101065403 A | |
| US 3510443 A | 05 May 1970 | GB 1176364 A claims, examples | |
| US 3341491 A | 12 Sep. 1967 | GB 1016377 A claims, examples | |
| JP 2010-18716 A | 28 Jan. 2010 | (Family: none) | |
| WO 01/016227 A1 | 08 Mar. 2001 | EP 1198504 A1 tables 1, 3, 4 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004500471 W
- JP 63023942 A
- JP 2009138148 A
- JP 2004091505 A
- JP 2000190704 A
- JP 2006328310 A
- JP 2009040898 A
- JP 2006249361 A
- JP 2019034798 A


### Non-patent literature cited in the description


- Gomu Kogyo Binran (Rubber Industry Handbook). 210-211
- Gomu Kogyo Binran (Rubber Industry Handbook). 494
- Gomu Kogyo Binran (Rubber Industry Handbook). The Society of Rubber Science and Technology, 20 January 1994
- Gomu Kogyo Binran (Rubber Industry Handbook). 436-443
- Gomu Kogyo Binran (Rubber Industry Handbook). 446-449